(19) Europäisches Patentamt
European Patent Office
Office européen des brevets

(11) **EP 4 190 342 A1**

(12) **EUROPEAN PATENT APPLICATION**
published in accordance with Art. 153(4) EPC

(43) Date of publication:
**07.06.2023 Bulletin 2023/23**

(21) Application number: **21848960.7**

(22) Date of filing: **29.07.2021**

(51) International Patent Classification (IPC):
*A61K 36/71* (2006.01)          *A61K 36/484* (2006.01)
*A61K 36/284* (2006.01)          *A61K 36/233* (2006.01)
*A61K 36/232* (2006.01)          *A61K 36/076* (2006.01)
*A61P 25/24* (2006.01)

(52) Cooperative Patent Classification (CPC):
A61K 36/076; A61K 36/232; A61K 36/233;
A61K 36/284; A61K 36/484; A61K 36/65;
A61K 36/71; A61P 25/00; A61P 25/24

(86) International application number:
**PCT/CN2021/109326**

(87) International publication number:
**WO 2022/022648 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.07.2020 CN 202010747978**

(71) Applicant: **Tasly Pharmaceutical Group Co., Ltd.
Tianjin 300410 (CN)**

(72) Inventors:
• **YAN, Kaijing**
  **Tianjin 300410 (CN)**

• **YE, Zhengliang**
  **Tianjin 300410 (CN)**
• **ZHANG, Shunnan**
  **Tianjin 300410 (CN)**
• **ZHANG, Wensheng**
  **Tianjin 300410 (CN)**
• **ZHENG, Yongfeng**
  **Tianjin 300410 (CN)**
• **FAN, Lijun**
  **Tianjin 300410 (CN)**
• **DU, Peng**
  **Tianjin 300410 (CN)**

(74) Representative: **Schröer, Gernot H.
Meissner Bolte Patentanwälte
Rechtsanwälte Partnerschaft mbB
Bankgasse 3
90402 Nürnberg (DE)**

(54) **TRADITIONAL CHINESE MEDICINE COMPOSITION HAVING MENTAL RELIEF AND ANTIDEPRESSANT EFFECTS AND PREPARATION METHOD THEREFOR**

(57)     A traditional Chinese medicine composition having mental relief and antidepressant effects and a preparation method therefor. The traditional Chinese medicine composition has two formulas: formula 1, comprising in parts by weight: 10 to 30 parts of Chinese Thorowax Root *(Bupleuri Radix),* 10 to 30 parts of White Peony Root *(Paeoniae Alba Radix),* 15 to 30 parts of Atractylodis Macrocephalae Rhizoma (processed with bran), 10 to 30 parts of Chinese Angelica *(Angelicas Sinensis Radix),* and 15 to 24 parts of prepared liauorice root *(Glycyrrhizae Radix et Rhizon Praeparata cum Melle);* and formula 2, comprising in parts by weight: 10 to 30 parts of Chinese Thorowax Root *(Bupleuri Radix),* 10 to 30 parts of White Peony Root *(Paeoniae Alba Radix),* 15 to 30 parts of Atractylodis Macrocephalae Rhizoma (processed with bran), 10 to 30 parts of Chinese Angelica *(Angelicas Sinensis Radix),* 10 to 30 parts of Indian Bread *(Poria),* and 15 to 24 parts of prepared liauorice root *(Glycyrrhizae Radix et Rhizon Praeparata cum Melle).*

Figure 1

**Description**

Technical Field

The present application belongs to the field of traditional Chinese medicine, and particularly relates to, but is not limited to, a traditional Chinese medicine composition having mental relief and antidepressant effects, and a preparation method and application thereof.

Background

Depression is a common mental disease with depression as the main symptom caused by various reasons. With the development of society and the increase of pressure in people's life and work, people's mental pressure is also increasing, resulting in an upward trend in the incidence of depression, and its harmfulness has attracted more and more attention in the medical and health sector. There are many inducements and complicated pathogenesis of depression, which leads to a low recognition rate of depression diagnosis.

At present, the drugs used to treat depression in the market are mainly chemical drugs, but the antidepressant effect is not ideal, and there are some problems such as high price and severe side effects. In contrast, traditional Chinese herbal medicine has the advantages such as low toxic side effects, few adverse reactions and long-lasting effects. Therefore, finding ideal antidepressants from the traditional Chinese herbal medicine is a research hotspot in recent years.

*Xiaoyao Powder* comes from *Taiping Huimin Hejiju Prescription* in Song Dynasty. Experimental studies and clinical practice have proved that it has a definite antidepressant effect. *Xiaoyao Powder* is composed of eight traditional Chinese medicines: Chinese Thorowax Root *(Bupleuri Radix),* Chinese Angelica *(Angelicae Sinensis Radix),* Rhizoma atractylodis macrocephalae, White Peony Root *(Paeoniae Alba Radix),* Indian Bread *(Poria)(Poria),* Peppermint, Ginger, and licorice root. It is derived from *Sini Powder* and has the effects of soothing the liver and relieving depression, invigorating the spleen and harmonizing the nutrient. It is a commonly used prescription for harmonizing the liver and spleen. According to the distribution of sovereign, minister, assistant and guiding medicinals, Chinese Thorowax Root *(Bupleuri Radix),* Chinese Angelica *(Angelicae Sinensis Radix)* and White Peony Root *(Paeoniae Alba Radix)* are the sovereign medicinals, Rhizoma atractylodis macrocephalae, Indian Bread *(Poria)* and Ginger are the minister medicinals, peppermint is the assistant medicinal, and prepared liauorice root *(Glycyrrhizae Radix et Rhizon Praeparata cum Melle)(Glycyrrhizae Radix et Rhizon Praeparata cum Melle)* is the guiding medicinal. The eight medicinal materials of *Xiaoyao Power* has different pharmacological effects, wherein Chinese Thorowax Root *(Bupleuri Radix)* is mainly used to soothe the liver and relieve depression, Chinese Angelica *(Angelicae Sinensis Radix)* and White Peony Root *(Paeoniae Alba Radix)* are used to nourish the blood and soften the liver, and these three medicinal materials cooperate with each other, and they can both nourish and harmonize the liver. Rhizoma atractylodis macrocephalae, Indian Bread *(Poria)* and Ginger can strengthen the spleen when used together. Peppermint can help Chinese Thorowax Root *(Bupleuri Radix)* to disperse the stagnation, and prepared liauorice root *(Glycyrrhizae Radix et Rhizon Praeparata cum Melle)* can replenish *qi* and tonify the middle energizer, thus blending various medicines.

Chinese patents CN108653405A and CN107625813A disclose a traditional Chinese medicine composition consisting of six traditional Chinese medicines, "the components of the traditional Chinese medicine being, in parts by mass, 20-60 parts of Chinese Thorowax Root *(Bupleuri Radix),* 20-60 parts of Chinese Angelica *(Angelicae Sinensis Radix),* 20-60 parts of Atractylodis Macrocephalae Rhizoma (processed with bran), 20-60 parts of White Peony Root *(Paeoniae Alba Radix),* 8-20 parts of Peppermint and 8-20 parts of prepared liauorice root *(Glycyrrhizae Radix et Rhizon Praeparata cum Melle)*; and preparation method thereof, which comprises the following steps: 1) to the components of the medicinal materials, adding 60-90% ethanol having weight of 6-10 times the weight of the components of the medicinal materials, extracting for 2 times, each time for 1-3 h, filtering, combining the filtrates, and concentrating to obtain alcohol-extracted thin paste; 2) to the medicine residue, adding water having 6-10 times the weight the medicine residue, extracting for 2 times, each time for 1-3h, filtering, combining the filtrates, concentrating to obtain water-extracted thin paste, then adding ethanol into the water-extract thin paste to make the alcohol content reach 60-80%, standing for 24h, taking the supernatant, and concentrating to obtain a thin paste; 3) combing the thin paste with the alcohol-extracted thin paste, mixing them evenly, and then concentrating them into a thick paste, drying it under reduced pressure by microwave, and pulverizing and screening with 80 mesh sieve to obtain the traditional Chinese medicine extract".

Chinese patent CN101732427A discloses a pharmaceutical composition consisting of "Chinese Thorowax Root *(Bupleuri Radix)* fine powder, Chinese Angelica *(Angelicae Sinensis Radix)* extract, White Peony Root *(Paeoniae Alba Radix)* extract, Rhizoma atractylodis macrocephalae extract, Indian Bread *(Poria)* extract, licorice root extract and Peppermint extract".

Chinese patent CN102813906A discloses a pharmaceutical composition consisting of "Chinese Thorowax Root *(Bupleuri Radix),* Chinese Angelica *(Angelicae Sinensis Radix),* stir-fried Rhizoma Atractylodis Macrocephalae, prepared liauorice root *(Glycyrrhizae Radix et Rhizon Praeparata cum Melle)*, Peppermint and dried ginger".

Chinese patent CN104644776A discloses a traditional Chinese medicine powder for treating irregular menstruation prepared from "Chinese Thorowax Root *(Bupleuri Radix)*, Chinese Angelica *(Angelicae Sinensis Radix)*, Indian Bread *(Poria)*, White Peony Root *(Paeoniae Alba Radix)*, Rhizoma atractylodis macrocephalae and licorice root".

Chinese patent CN108653405A discloses a preparation method which comprises the following steps of: firstly combining alcohol extracts to obtain an alcohol-extracted thin paste, and then water-extract thin paste, adding alcohol into the water-extracted thin paste to obtain a thin paste, and combining the alcohol-extracted thin paste and the thin paste obtained for the second time. Chinese Thorowax Root *(Bupleuri Radix)*, Chinese Angelica *(Angelicae Sinensis Radix)*, White Peony Root *(Paeoniae Alba Radix)*, Rhizoma atractylodis macrocephalae and licorice root in the recipe of *Xiaoyao Powder* have volatile components or volatile oils, and volatile oils are extracted by methods such as a steam distillation method, which is also widely used in the preparation of *Xiaoyao* products.

Chinese patent CN1017342427A discloses a preparation method of combining other components except Chinese Thorowax Root *(Bupleuri Radix)* in the prescription and extracting with water and retaining volatile matters, adding ethanol into the water-extracted thick paste and filtering twice, and combining the filtrate with volatile matters and Chinese Thorowax Root *(Bupleuri Radix)* fine powder.

Chinese patent publication CN104189833A discloses a preparation method of a tablet, which comprises the following steps: 1) distilling four Chinese medicine materials of Chinese Thorowax Root *(Bupleuri Radix)*, Chinese Angelica *(Angelicae Sinensis Radix)*, fried Rhizoma atractylodis macrocephalae and Peppermint by steam distillation method to obtain a volatile oil; 2) treating the volatile oil with cyclodextrin to obtain a clathrate; 3) combing White Peony Root *(Paeoniae Alba Radix)*, prepared liauorice root *(Glycyrrhizae Radix et Rhizon Praeparata cum Melle)*, Indian Bread *(Poria)* and the medicine residues extracted in step 1) and extracting with water, and adding the clathrate of volatile oil, so as to prepare the tablet.

Chinese patent publication CN102908600A discloses a preparation method of a pill, which comprises the following steps: 1) extracting volatile oils from Chinese Thorowax Root *(Bupleuri Radix)*, Ginger, Peppermint and Chinese Angelica *(Angelicae Sinensis Radix)*; 2) extracting the medicine residues after extraction and stir-fried Rhizoma atractylodis macrocephalae and Indian Bread *(Poria)* with water, and concentrating to obtain a thick paste; 3) crushing White Peony Root *(Paeoniae Alba Radix)*, one quarter equivalent of prepared liauorice root *(Glycyrrhizae Radix et Rhizon Praeparata cum Melle)* and the remaining equivalent of Chinese Angelica *(Angelicae Sinensis Radix)* into medicine powder; 4) preparing the remaining amount of prepared liauorice root *(Glycyrrhizae Radix et Rhizon Praeparata cum Melle)* into a concentrate; and 5) combining them to prepare the pill.

Summary

The following is a summary of the subject matter described in detail herein. This summary is not intended to limit the scope of protection of the claims.

The present application provides a traditional Chinese medicine composition having mental relief and antidepressant effects and a preparation method and application thereof.

The inventors of the present application have made a careful study on the formulation of the prior art, applied modern science and technology to study the antidepressant mechanism of *Xiaoyao Powder* and its similar formulation, and screened out the formulation with more superior depression treatment effect from *Xiaoyao Powder* and its similar formulation, and simultaneously carries out new exploration on the preparation method in order to reduce the cost, simplify the process and optimize the quality control, thereby completing the present invention.

The present application has the following characteristics: (1) after studying various prescriptions, the inventors of the present application have found that the selected formulation is simpler and lower in cost, and achieves more superior antidepressant effects in the antidepressant model of rat compared with *Xiaoyao Pill* whole formulation (seven medicinal materials, Chinese Pharmacopoeia, 2015 edition, pages 1354-1355) or a similar formulation with peppermint added (six medicinalmaterials) (CN108653405A and CN107625813A); (2) only water was used as a solvent for extraction in the preparation process, so that the preparation process is simpler, safer and more controllable, and the cost is lower; (3) the inventors of the present application have adopted a grouped extraction process, which avoided the interference of other ingredients on the quantification of ferulic acid in Chinese Angelica *(Angelicae Sinensis Radix)*, and meanwhile, each medicinal material can be subjected to quality control after grouped extraction, and mutual interference can be avoided. Specifically, Chinese Thorowax Root *(Bupleuri Radix)(Bupleuri Radix)* is separated from Chinese Angelica *(Angelicae Sinensis Radix)* to avoid the interference of Chinese Thorowax Root *(Bupleuri Radix)* components on the quantification of ferulic acid in Chinese Angelica *(Angelicae Sinensis Radix)*; at least one stable index component is contained in the extracts in each group so as to characterize the process stability of the process from extraction to formulation; the Atractylodis Macrocephalae Rhizoma (processed with bran) and Chinese Angelica *(Angelicae Sinensis Radix)* are divided into one group since they contain volatile components, and aromatic water is collected during the extraction process. The aromatic water is added into the extract at the endpoint of concentrating and collecting the paste, so that the whole extract is close to the water decoction components from the traditional application.

In a first aspect, the present application provides a traditional Chinese medicine composition having mental relief and antidepressant effects, which is prepared from the formulation of traditional Chinese medicine raw materials in parts by weight consisting of Chinese Thorowax Root *(Bupleuri Radix)*, White Peony Root *(Paeoniae Alba Radix)*, Atractylodis Macrocephalae Rhizoma (processed with bran), Chinese Angelica *(Angelicae Sinensis Radix)*, prepared liauorice root *(Glycyrrhizae Radix et Rhizon Praeparata cum Melle)*, and optionally with or without Indian Bread *(Poria)*, wherein the formulation without Indian Bread *(Poria)* is formulation 1:

10-30 parts of Chinese Thorowax Root *(Bupleuri Radix)*, 10-30 parts of White Peony Root *(Paeoniae Alba Radix)*, 15-30 parts of Atractylodis Macrocephalae Rhizoma (processed with bran), 10-30 parts of Chinese Angelica *(Angelicae Sinensis Radix)* and 15-24 parts of prepared liauorice root *(Glycyrrhizae Radix et Rhizon Praeparata cum Melle)*;

wherein the formulation with Indian Bread *(Poria)* is formulation 2:

10-30 parts of Chinese Thorowax Root *(Bupleuri Radix)*, 10-30 parts of White Peony Root *(Paeoniae Alba Radix)*, 15-30 parts of Atractylodis Macrocephalae Rhizoma (processed with bran), 10-30 parts of Chinese Angelica *(Angelicae Sinensis Radix)*, 10-30 parts of Indian Bread *(Poria)* and 15-24 parts of prepared liauorice root *(Glycyrrhizae Radix et Rhizon Praeparata cum Melle)*.

In a second aspect, the present application provides a preparation method of the traditional Chinese medicine composition described above, which comprises the following steps: weighing the medicinal materials according to the ratio of the formulation, adding water of 4-7 times the weight of the medicinal materials, extracting for 1-3 times, each time for 1-3 h, filtering after extraction, combining the filtrates from the two extractions, standing for 4 h or above, taking the supernatant, and concentrating the supernatant to obtain an extract; taking the extract as the active component of the medicine, and adding medicinal excipients when necessary, to prepare into a traditional Chinese medicine composition pharmaceutical formulation.

In a third aspect, the present application also provides a traditional Chinese medicine composition pharmaceutical formulation (hereinafter referred to as a traditional Chinese medicine formulation composition), the traditional Chinese medicine formulation composition is an oral preparation or an injection. In some exemplary embodiments, the traditional Chinese medicine composition may be in any orally administrable pharmaceutical form, such as tablets, sugar-coated tablets, thin film-coated tablets, enteric-coated tablets, capsules, hard capsules, soft capsules, oral liquids, oral preparations, granules, pills, powders, ointments, pills of immortality, suspensions, powders, solutions, injections, suppositories, ointments, plasters, creams, sprays, drops or patches.

According to the present application, through the research on the antidepressant mechanism, the prescription of the formulation and the preparation process of the *Xiaoyao Powder* and the similar formulation, it can be seen that the antidepressant efficacy of the *Xiaoyao Powder* is different with different formulations and different preparation processes, and the compositions of the obtained active components which exert the antidepressant efficacy are quite different. According to the specific medicinal materials and proportions, the present application adopts a specific extraction process to obtain different compositions having the antidepressant effects.

Other aspects will become apparent upon reading and understanding the drawings and detailed description.

Brief Description of Drawings

The drawings are used to provide an understanding of the technical schemes of the present application, and constitute a part of the specification. They are used to explain the technical schemes of the present application together with the embodiments of the present application, and do not constitute a limitation to the technical schemes of the present application.

FIG. 1: The specificity of the analysis method for the content of ferulic acid extracted from the combined five medicinal materials is unqualified, wherein E03 is Example 14 of the present application.

FIG. 2: The specificity of the analytical method for the content of ferulic acid in *Xiaoyao Dangbaigan* extract is qualified.

FIG. 3: Curve of change in the content in glycyrrhizic acid.

Detailed Description

In the first aspect, the present application provides a traditional Chinese medicine composition having mental relief and antidepressant effects, which is prepared from the formulation of traditional Chinese medicine raw materials in parts by weight consisting of Chinese Thorowax Root *(Bupleuri Radix)*, White Peony Root *(Paeoniae Alba Radix)*, Atractylodis Macrocephalae Rhizoma (processed with bran), Chinese Angelica *(Angelicae Sinensis Radix)*, prepared liauorice root *(Glycyrrhizae Radix et Rhizon Praeparata cum Melle)*, and optionally with or without Indian Bread *(Poria)*, wherein the formulation without Indian Bread *(Poria)* is formulation 1:

10-30 parts of Chinese Thorowax Root *(Bupleuri Radix)*, 10-30 parts of White Peony Root *(Paeoniae Alba Radix)*, 15-30 parts of Atractylodis Macrocephalae Rhizoma (processed with bran), 10-30 parts of Chinese Angelica *(Angelicae Sinensis Radix)*, and 15-24 parts of prepared liauorice root *(Glycyrrhizae Radix et Rhizon Praeparata cum Melle)*;

wherein the formulation with Indian Bread *(Poria)* is formulation 2:
10-30 parts of Chinese Thorowax Root *(Bupleuri Radix)*, 10-30 parts of White Peony Root *(Paeoniae Alba Radix)*, 15-30 parts of Atractylodis Macrocephalae Rhizoma (processed with bran), 10-30 parts of Chinese Angelica *(Angelicae Sinensis Radix)*, 10-30 parts of Indian Bread *(Poria)*, and 15-24 parts of prepared liauorice root *(Glycyrrhizae Radix et Rhizon Praeparata cum Melle)*.

In some exemplary embodiments, the formulation 1 is, in parts by weight, 15-25 parts of Chinese Thorowax Root *(Bupleuri Radix)*, 15-25 parts of White Peony Root *(Paeoniae Alba Radix)*, 15-25 parts of Atractylodis Macrocephalae Rhizoma (processed with bran), 15-25 parts of Chinese Angelica *(Angelicae Sinensis Radix)* and 15-25 parts of prepared liauorice root *(Glycyrrhizae Radix et Rhizon Praeparata cum Melle)*; the formulation 2 is, in parts by weight, 15-25 parts of Chinese Thorowax Root *(Bupleuri Radix)*, 15-25 parts of White Peony Root *(Paeoniae Alba Radix)*, 15-25 parts of Atractylodis Macrocephalae Rhizoma (processed with bran), 15-25 parts of Chinese Angelica *(Angelicae Sinensis Radix)*, 15-25 parts of Indian Bread *(Poria)*, and 15-20 parts of prepared liauorice root *(Glycyrrhizae Radix et Rhizon Praeparata cum Melle)*.

In some exemplary embodiments, the formulation 1 is, in parts by weight, 20 parts of Chinese Thorowax Root *(Bupleuri Radix)*, 20 parts of White Peony Root *(Paeoniae Alba Radix)*, 20 parts of Atractylodis Macrocephalae Rhizoma (processed with bran), 20 parts of Chinese Angelica *(Angelicae Sinensis Radix)*, and 16 parts of prepared liauorice root *(Glycyrrhizae Radix et Rhizon Praeparata cum Melle)*; the formulation 2 is, in parts by weight, 20 parts of Chinese Thorowax Root *(Bupleuri Radix)*, 20 parts of White Peony Root *(Paeoniae Alba Radix)*, 20 parts of Atractylodis Macrocephalae Rhizoma (processed with bran), 20 parts of Chinese Angelica *(Angelicae Sinensis Radix)*, 20 parts of Indian Bread *(Poria)*, and 16 parts of prepared liauorice root *(Glycyrrhizae Radix et Rhizon Praeparata cum Melle)*.

In some exemplary embodiments, the traditional Chinese medicine composition of the formulation 1 includes 9 parts of *Xiaoyao Chaibai* extract by dry weight, which is extracted from 10-30 part of Chinese Thorowax Root *(Bupleuri Radix)* and 10-30 part of White Peony Root *(Paeoniae Alba Radix)*; 32 parts of *Xiaoyao Dangbaigan* extract by dry weight, which is extracted from 15-30 parts of Atractylodis Macrocephalae Rhizoma (processed with bran), 10-30 parts of Chinese Angelica *(Angelicae Sinensis Radix)* and 15-24 parts of prepared liauorice root *(Glycyrrhizae Radix et Rhizon Praeparata cum Melle)*; wherein, the dry content of saikosaponin B2 is 0.63-10.0 mg/g, and the dry content of paeoniflorin is 9.4-115.9 mg/g in the *Xiaoyao Chaibai* extract; the dry content of glycyrrhizic acid is 1.4 ~26.1mg/g, the dry content of ferulic acid is 0.13-0.91mg/g, and the dry content of atractylenolide III is 0.0038-0.30mg/g in the *Xiaoyao Dangbaigan* extract.

In some exemplary embodiment, the dry content of saikosaponin B2 is 0.94~6.8 mg/g, and the dry content of Paeoniflorin is 17.6~110.9mg/g in the *Xiaoyao Chaibai* extract; the dry content of glycyrrhizic acid is 6.9-26.1 mg/g, the dry content of ferulic acid is 0.16~0.79 mg/g, and the dry content of atractylenolide III is 0.0047~0.25 mg/g in the *Xiaoyao Dangbaigan* extract.

The dry weight mentioned in the present application refers to the weight after being converted into a dried sample, that is, the weight after the sample is dried. In a second aspect, the present application provides a preparation method of the traditional Chinese medicine composition as described above, which comprises the following steps: weighing the medicinal materials according to the ratio of the formulation, adding water of 4-7 times the weight of the medicinal materials, extracting for 1-3 times, each time for 1-3 h, filtering after extraction, combining the filtrates from the two extractions, standing for 4h or above, taking the supernatant, and concentrating the supernatant to obtain an extract; taking the extract as the active component of the medicine, and adding medicinal excipients when necessary, to prepare into a traditional Chinese medicine composition pharmaceutical formulation.

In some exemplary embodiments, the preparation method comprises the steps of: weighing the medicinal materials according to the ratio of the formulation, adding water of 6-7 times of the medicinal materials, extracting for 2-3h, filtering, adding water of 5-6 times weight into the medicine residue, extracting for 1h, filtering, combining the filtrates from the two extractions, standing for 4h or above, taking the supernatant, concentrating, controlling the concentration end point at a sugar degree of 65%-70%, taking the extract as the active component of the medicine and adding medicinal excipients when necessary, to prepare into a traditional Chinese medicine composition pharmaceutical formulation.

In some exemplary embodiments, the preparation method of the traditional Chinese medicine composition of formulation 1 comprises the steps of:

(1) extraction of *Xiaoyao Chaibai* extract: adding water of 4-7 times weight into Chinese Thorowax Root *(Bupleuri Radix)* and White Peony Root *(Paeoniae Alba Radix)* medicinal materials, extracting for 1-3 times, each time for 1-3 h, filtering after extraction, combining the filtrates from the two extractions, standing for 4h or above, taking the supernatant, and concentrating to obtain the *Xiaoyao Chaibai* extract;

(2) extraction of the *Xiaoyao Dangbaigan* extract: adding water of 4-7 times weight into Chinese Angelica *(Angelicae Sinensis Radix)*, Atractylodis Macrocephalae Rhizoma (processed with bran) and prepared liauorice root *(Glycyrrhizae Radix et Rhizon Praeparata cum Melle)* medicine materials, extracting for 1-3 times, each time for 1-3 h, collecting the volatile oil and aromatic water after the extraction, filtering the extracted solution after the extraction, combining the extracted solutions from the two extractions, standing for 4 h or above, taking the supernatant, concentrating, adding the collected volatile oil and aromatic water, and concentrating again to obtain the *Xiaoyao Dangbaigan* extract;

(3) taking and evenly mixing the *Xiaoyao Chaibai* extract and the *Xiaoyao Dangbaigan* extract according to the proportion, taking the *Xiaoyao Chaibai* extract and the *Xiaoyao Dangbaigan* extract as active medicinal components, and adding medicinal excipients when necessary, to prepare into to a traditional Chinese medicine composition pharmaceutical formulation.

In some exemplary embodiments, the preparation method of the traditional Chinese medicine composition of formulation 1 comprises the steps of:

(1) extraction of *Xiaoyao Chaibai* extract: adding water of 6 times weight into Chinese Thorowax Root *(Bupleuri Radix)* and White Peony Root *(Paeoniae Alba Radix)* medicinal materials, extracting for 2h, filtering, adding water of 5 times weight into the medicine residue, extracting for 1h, filtering, combining the filtrates from the two extractions, standing for 4h or above, taking the supernatant, concentrating, and controlling the density of the concentration endpoint to be 1.26-1.28 to obtain the *Xiaoyao Chaibai* extract

(2) the extraction of the *Xiaoyao Dangbaigan* extract: adding water of 6 times weight into Chinese Angelica *(Angelicae Sinensis Radix)*, Atractylodis Macrocephalae Rhizoma (processed with bran) and prepared liauorice root *(Glycyrrhizae Radix et Rhizon Praeparata cum Melle)* medicinal materials, extracting for 2h, filtering, adding water of 5 times weight into the medicine residues, extracting for 1h, collecting volatile oil and aromatic water after extraction, filtering the extracted solution, combining the extracted solutions from the two extractions, standing for 4h or above, taking the supernatant, concentrating, concentrating to an endpoint, adding the collected volatile oil and aromatic water, concentrating again to an endpoint, and controlling the density of the concentration endpoint to be 1.29-1.31.

(3) taking and evenly mixing the *Xiaoyao Chaibai* extract and the *Xiaoyao Dangbaigan* extract according to the proportion, taking the *Xiaoyao Chaibai* extract and the *Xiaoyao Dangbaigan* extract as active medicinal components, and adding medicinal excipients when necessary, to prepare into a traditional Chinese medicine composition pharmaceutical formulation.

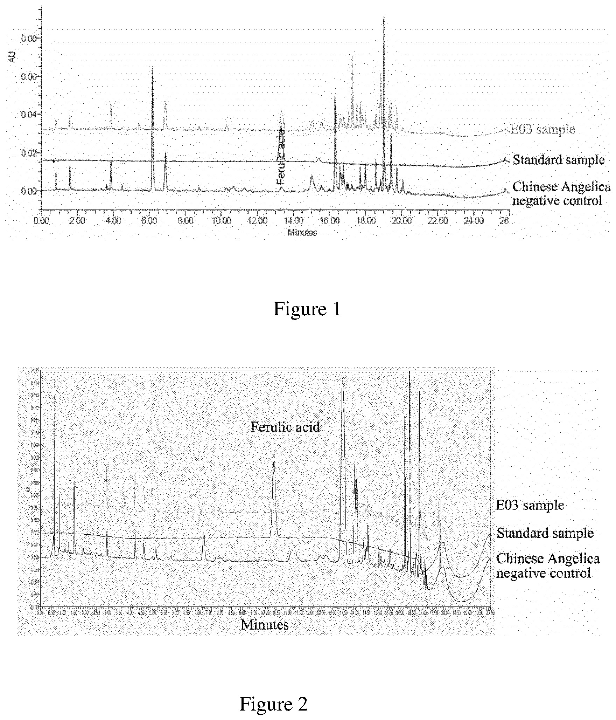

The inventors of the present application further found that the quantification of ferulic acid ingredient in Chinese Angelica *(Angelicae Sinensis Radix)* was interfered by some components of Chinese Thorowax Root *(Bupleuri Radix)*, and the specificity was unqualified when the remaining five medicinal materials were combined and extracted with water after peppermint and Indian Bread *(Poria)* were reduced from the prescription, as shown in FIG. 1. Considering that the ferulic acid ingredient in Chinese Angelica *(Angelicae Sinensis Radix)* can be quantified and is relatively stable, the inventors have designed a good extraction process, and the interference of other components on the quantification of ferulic acid in Chinese Angelica *(Angelicae Sinensis Radix)* was avoided by grouped extraction, as shown in FIG. 2. Grouped extraction is performed and the extracts are used as medicine, not only the efficacy of the medicine is not reduced, the quality thereof is effectively controlled.

Saikosaponin A and saikosaponin D can be detected in Chinese Thorowax Root *(Bupleuri Radix)* medicinal material, and hydrolysis has occurred during the extraction process, and saikosaponin D was converted into B2. Saikosaponin B2 can be detected in the extract and has the highest dry content.

In a third aspect, the present application also provides a traditional Chinese medicine composition pharmaceutical formulation (abbreviated as a traditional Chinese medicine formulation composition), the traditional Chinese medicine formulation composition can be an oral preparation or an injection.

Wherein the oral preparation is selected from one of capsule, tablet, dripping pill, granule, concentrated pill, oral liquid and mixture.

Wherein the injection is selected from one of injection solution, lyophilized powder injection and water injection.

In some exemplary embodiments, the traditional Chinese medicine formulationcomposition of the present application is any available pharmaceutical form, such as: tablets, sugar-coated tablet, thin film-coated tablet, enteric-coated tablet, capsule, hard capsule, soft capsule, oral liquids, oral preparations, granules, granules, pills, powders, ointments, pills of immortality, suspensions, powders, solutions, injections, suppositories, ointments, plasters, creams, sprays, drops or patches.

In some exemplary embodiments, the traditional Chinese medicine formulation composition of the present application is in the form of a unit dose pharmaceutical formulation.

When the traditional Chinese medicine formulation composition of the present application is prepared into medicaments, the medicament in unit dose can contain 0.1-1000 mg of the traditional Chinese medicine composition of the present application, and the rest is the pharmaceutically acceptable excipients. The pharmaceutically acceptable excipients can be 0.01-99.99% of the total weight of the preparation by weight.

When the traditional Chinese medicine formulation is used, the dosage is determined according to the patient's condition, such as 1-3 times a day, 1-20 tablets at a time, etc.

The formulation for oral administration of the traditional Chinese medicine formulation composition of the present application can contain commonly used pharmaceutical excipients, such as, but not limited to, binders, fillers, diluents, compressed tablets, lubricants, disintegrants, colorants, flavoring agents and wetting agents, and the tablets can be coated if necessary.

Suitable fillers include cellulose, mannitol, lactose, and other similar fillers. Suitable disintegrants include starch, polyvinylpyrrolidone, and starch derivatives such as sodium starch glycolate. Suitable lubricants include, for example, magnesium stearate. Suitable pharmaceutically acceptable wetting agents include sodium lauryl sulfate.

The traditional Chinese medicine formulation composition of the present application can be prepared into solid oral composition by common methods such as mixing, filling and tableting and the like. The active substance can be distributed throughout those compositions using a large amount of fillers by repeated mixing.

The oral liquid formulation can be, for example, in the form of an aqueous or oily suspension, solution, emulsion, syrup or elixir, or can be a dried product which can be compounded with water or other suitable carrier prior to use. Such liquid formulations may contain conventional additives, such as suspending agents such as sorbitol, syrup, methylcellulose, gelatin, hydroxyethyl cellulose, carboxymethyl cellulose, aluminum stearate gel or hydrogenated edible fat, emulsifying agents such as lecithin, sorbitan monooleate or gum arabic; non-aqueous carriers (which may include edible oils) such as almond oil, fractionated coconut oil, oily esters such as esters of glycerol, propylene glycol or ethanol; preservatives such as methyl p-hydroxybenzoate or propyl p-hydroxybenzoate or sorbic acid and, if desired, may contain conventional flavoring agents or coloring agents.

For injections, the prepared liquid unit dosage form contains the active substance of the present application and a sterile carrier. Depending on the carrier and concentration, the active substance can be suspended or dissolved. The solution is usually prepared by dissolving the active substance in a carrier, filtering and sterilizing, and then filling it into a suitable vial or ampoule, and then sealing. Excipients such as a local anesthetic, preservative and buffering agent can also be dissolved in this carrier. In order to improve its stability, the composition can be frozen after being filled into a vial, and the water can be removed under vacuum.

The present application is further described below with reference to specific examples.

Example 1

20 kg of Chinese Thorowax Root *(Bupleuri Radix)* and 20 kg of White Peony Root *(Paeoniae Alba Radix)* were weighed according to the formulation ratio, and water of 6 times weight was added, extracted for 2 h, filtrated, and water of 5 times weight was added into the medicine residue, extracted for 1h, filtrated, and the filtrates from the two extractions were combined and allowed to stand for 4 h or above under ambient temperature conditions. The supernatant was taken, and concentrated to a relative density of 1.27 to obtain the *Xiaoyao Chaibai* extract. The dry content of saikosaponin B2 was 2.7 mg/g, and the dry content of paeoniflorin was 42.1 mg/g therein.

Example 2

20 kg of Chinese Thorowax Root *(Bupleuri Radix)* and 20 kg of White Peony Root *(Paeoniae Alba Radix)* were weighed according to the formulation ratio, and water of 7 times weight was added, extracted for 3 h, filtrated, and water of 4 times weight was added into the medicine residue, extracted for 1h, filtrated, and the filtrates from the two extractions were combined and allowed to stand for 4 h or above under ambient temperature conditions. The supernatant was taken, and concentrated to a relative density of 1.27 to obtain the *Xiaoyao Chaibai* extract. The dry content of saikosaponin B2 was 2.9 mg/g, and the dry content of paeoniflorin was 40.8 mg/g therein.

Example 3

15 kg of Chinese Thorowax Root *(Bupleuri Radix)* and 25 kg of White Peony Root *(Paeoniae Alba Radix)* were weighed according to the formulation ratio, and water of 6 times weight was added, extracted for 2 h, filtrated, and water of 5 times weight of water was added into the medicine residue, extracted for 1h, filtrated, and the filtrates from the two extractions were combined and allowed to stand for 4 h or above under ambient temperature conditions. The supernatant was taken, and concentrated to a relative density of 1.27 to obtain the *Xiaoyao Chaibai* extract. The dry content of saikosaponin B2 was 2.1 mg/g, and the dry content of paeoniflorin was 54.2 mg/g therein.

Example 4

25 kg of Chinese Thorowax Root *(Bupleuri Radix)* and 15 kg of White Peony Root *(Paeoniae Alba Radix)* were weighed according to the formulation ratio, and water of 7 times weight was added, extracted for 3 h, filtrated, and water of 4 times weight was added into the medicine residue, extracted for 1h, filtrated, and the filtrates from the two extractions were combined and allowed to stand for 4 h or above under ambient temperature conditions. The supernatant was taken, and concentrated to a relative density of 1.27 to obtain the *Xiaoyao Chaibai* extract. The dry content of saikosaponin B2 was 3.1 mg/g, and the dry content of paeoniflorin was 30.9 mg/g therein.

Example 5

30 kg of Chinese Thorowax Root *(Bupleuri Radix)* and 10 kg of White Peony Root *(Paeoniae Alba Radix)* were weighed according to the formulation ratio, and water of 6 times weight was added, extracted for 2 h, filtrated, and water of 5 times weight was added into the medicine residue, extracted for 1h, filtrated, and the filtrates from the two extractions were combined and allowed to stand for 4 h or above under ambient temperature conditions. The supernatant was taken, and concentrated to a relative density of 1.27 to obtain the *Xiaoyao Chaibai* extract. The dry content of saikosaponin B was 23.7 mg/g, and the dry content of paeoniflorin was 22.2 mg/g therein.

Example 6

10 kg of Chinese Thorowax Root *(Bupleuri Radix)* and 30 kg of White Peony Root *(Paeoniae Alba Radix)* were weighed according to the formulation ratio, and water of 7 times weight was added, extracted for 3 h, filtrated, and water of 4 times weight was added into the medicine residue, extracted for 1h, filtrated, and the filtrates from the two extractions were combined and allowed to stand for 4 h or above under ambient temperature conditions. The supernatant was taken, and concentrated to a relative density of 1.27 to obtain the *Xiaoyao Chaibai* extract. The dry content of saikosaponin B was 21.4 mg/g, and the dry content of paeoniflorin was 65.70 mg/g therein.

Example 7

20 kg of Chinese Angelica *(Angelicae Sinensis Radix)*, 20 kg of Atractylodis Macrocephalae Rhizoma (processed with bran) and 16 kg of prepared liauorice root *(Glycyrrhizae Radix et Rhizon Praeparata cum Melle)* were weighed according to the formulation ratio, and water of 6 times weight was added, extracted for 2 h, filtrated, and water of 5 times weight was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken and concentrated to a relative density of 1.30. The collected volatile oil and aromatic water were added, and continued to concentrate to a relative density of 1.30 to obtain the *Xiaoyao Dangbaigan* extract, wherein the dry content of glycyrrhizic acid is 15.7 mg/g, the dry content of ferulic acid is 0.50 mg/g, and the dry content of atractylenolide III is 0.0.0078mg/g.

Example 8

20 kg of Chinese Angelica *(Angelicae Sinensis Radix)*, 20 kg of Atractylodis Macrocephalae Rhizoma (processed with bran) and 16 kg of prepared liauorice root *(Glycyrrhizae Radix et Rhizon Praeparata cum Melle)* were weighed according to the formulation ratio, and water of 7 times weight was added, extracted for 3 h, filtrated, and water of 4 times weight was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken and concentrated to a relative density of 1.30. The collected volatile oil and aromatic water were added, and continued to concentrate to a relative density of 1.30 to obtain the *Xiaoyao Dangbaigan* extract, wherein the dry content of glycyrrhizic acid is 15.4 mg/g, the dry content of ferulic acid is 0.48 mg/g, and the dry content of atractylenolide III is 0.0073mg/g.

Example 9

25 kg of Chinese Angelica *(Angelicae Sinensis Radix),* 15 kg of Atractylodis Macrocephalae Rhizoma (processed with bran) and 15 kg of prepared liauorice root *(Glycyrrhizae Radix et Rhizon Praeparata cum Melle)* were weighed according to the formulation ratio, and water of 6 times weight was added, extracted for 2 h, filtrated, and water of 5 times weight was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken, and concentrated to a relative density of 1.30. The collected volatile oil and aromatic water were added, and continued to concentrate to a relative density of 1.30 to obtain the *Xiaoyao Dangbaigan* extract, wherein the dry content of glycyrrhizic acid is 11.9 mg/g, the dry content of ferulic acid is 0.62 mg/g, and the dry content of atractylenolide III is 0.0058 mg/g.

Example 10

25 kg of Chinese Angelica *(Angelicae Sinensis Radix),* 15 kg of Atractylodis Macrocephalae Rhizoma (processed with bran) and 20 kg of prepared liauorice root *(Glycyrrhizae Radix et Rhizon Praeparata cum Melle)* were weighed according to the formulation ratio, and water of 7 times weight was added, extracted for 3 h, filtrated, and water of 4 times weight was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken and concentrated to a relative density of 1.30. The collected volatile oil and aromatic water were added, and continued to concentrate to arelative density of 1.30 to obtain the *Xiaoyao Dangbaigan* extract, wherein the dry content of glycyrrhizic acid is 18.8 mg/g, the dry content of ferulic acid is 0.59 mg/g, and the dry content of Atractylenolide III is 0.0055mg/g.

Example 11

15 kg of Chinese Angelica *(Angelicae Sinensis Radix),* 25 kg of Atractylodis Macrocephalae Rhizoma (processed with bran) and 18 kg of prepared liauorice root *(Glycyrrhizae Radix et Rhizon Praeparata cum Melle)* were weighed according to the formulation ratio, and water of 6 times weight was added, extracted for 2 h, filtrated, and water of 5 times weight of water was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken, and concentrated to a relative density of 1.30. The collected volatile oil and aromatic water were added, and continued to concentrate to a relative density of 1.30 to obtain the *Xiaoyao Dangbaigan* extract, wherein the dry content of glycyrrhizic acid is 16.9 mg/g, the dry content of ferulic acid is 0.36 mg/g, and the dry content of atractylenolide III is 0.0092 mg/g.

Example 12

30 kg of Chinese Angelica *(Angelicae Sinensis Radix),* 15 kg of Atractylodis Macrocephalae Rhizoma (processed with bran) and 24 kg of prepared liauorice root *(Glycyrrhizae Radix et Rhizon Praeparata cum Melle)* were weighed according to the formulation ratio, and water of 7 times weight was added, extracted for 3 h, filtrated, and water of 4 times weight was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken, and concentrated to a relative density of 1.30. The collected volatile oil and aromatic water were added, and continued to concentrate to a relative density of 1.30 to obtain the *Xiaoyao Dangbaigan* extract, wherein the dry content of glycyrrhizic acid is 21.4 mg/g, the dry content of ferulic acid is 0.67 mg/g, and the dry content of atractylenolide III is 0.0047 mg/g.

Example 13

10 kg of Chinese Angelica *(Angelicae Sinensis Radix),* 30 kg of Atractylodis Macrocephalae Rhizoma (processed with bran) and 15 kg of prepared liauorice root *(Glycyrrhizae Radix et Rhizon Praeparata cum Melle)* were weighed according to the formulation ratio, and water of 7 times weight was added, extracted for 3 h, filtrated, and water of 4 times weight was added into the medicine residue, extracted for 1h. Volatile oil and aromatic water were collected after extraction. The extracted solutions were filtered. The extracted solutions from the two extractions were combined, and allowed to stand for 4 h or above under ambient temperature conditions. The supernatant was taken, and concentrated to a relative density of 1.30. The collected volatile oil and aromatic water were added, and continued to concentrate to a relative density of 1.30 to obtain the *Xiaoyao Dangbaigan* extract, wherein the dry content of glycyrrhizic acid is 7.4 mg/g, the dry content of ferulic acid is 0.23 mg/g, and the dry content of atractylenolide III is 0.0154mg/g.

Example 14

9 kg (dry weight) of the *Xiaoyao Chaibai* extract from Example 1 and 32 kg (dry weight) of the *Xiaoyao Dangbaigan* extract from Example 7 were taken and mixed evenly to obtain the traditional Chinese medicine composition of the present application.

Example 15

The *Xiaoyao Chaibai* extract in any one of the Examples 1-6 and the *Xiaoyao Dangbaigan* extract in any one of the Examples 7-13 were taken and mixed evenly according to the dry weight ratio of 9:32 to obtain the traditional Chinese medicine composition of the present application.

Example 16

20 kg of Chinese Thorowax Root *(Bupleuri Radix),* 20 kg of White Peony Root *(Paeoniae Alba Radix),* 20 kg of Atractylodis Macrocephalae Rhizoma (processed with bran), 20 kg of Chinese Angelica *(Angelicae Sinensis Radix)* and 16 kg of prepared liauorice root *(Glycyrrhizae Radix et Rhizon Praeparata cum Melle).* The medicinal materials were weighed according to the formulation ratio, and water of 6 times weight was added, extracted for 2 h, filtrated, and water of 5 times weight was added into the medicine residue, extracted for 1h, and filtrated. The filtrates from the two extractions were combined, and allowed to stand for 4 h or above under ambient temperature conditions. The supernatant was taken, and the sugar content is controlled at 65%~70% at the concentration endpoint to obtain the traditional Chinese medicine extract.

Example 17

10 kg of Chinese Thorowax Root *(Bupleuri Radix),* 10 kg of White Peony Root *(Paeoniae Alba Radix),* 15 kg of Atractylodis Macrocephalae Rhizoma (processed with bran), 10 kg of Chinese Angelica *(Angelicae Sinensis Radix)* and 15 kg of prepared liauorice root *(Glycyrrhizae Radix et Rhizon Praeparata cum Melle).* It was prepared according to Example 16. The medicinal materials were weighed according to the formulation ratio, and water of 6 times weight was added, extracted for 2 h, filtrated, and water of 5 times weight was added into the medicine residue, extracted for 1h, and filtrated. The filtrates from the two extractions were combined, and allowed to stand for 4 h or above under ambient temperature conditions. The supernatant was taken, and the sugar content is controlled at 68%~72% at the concentration endpoint to obtain the traditional Chinese medicine extract.

Example 18

30 kg of Chinese Thorowax Root *(Bupleuri Radix),* 30 kg of White Peony Root *(Paeoniae Alba Radix),* 30 kg of Atractylodis Macrocephalae Rhizoma (processed with bran), 30 kg of Chinese Angelica *(Angelicae Sinensis Radix)* and 24 kg of prepared liauorice root *(Glycyrrhizae Radix et Rhizon Praeparata cum Melle).* The medicinal materials were weighed according to the formulation ratio, and water of 6 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 19

15 kg of Chinese Thorowax Root *(Bupleuri Radix),* 15 kg of White Peony Root *(Paeoniae Alba Radix),* 15 kg of Atractylodis Macrocephalae Rhizoma (processed with bran), 15 kg of Chinese Angelica *(Angelicae Sinensis Radix)* and 15 kg of prepared liauorice root *(Glycyrrhizae Radix et Rhizon Praeparata cum Melle).* The medicinal materials were weighed according to the formulation ratio, and water of 6 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 20

25 kg of Chinese Thorowax Root *(Bupleuri Radix)*, 25 kg of White Peony Root *(Paeoniae Alba Radix)*, 25 kg of Atractylodis Macrocephalae Rhizoma (processed with bran), 25 kg of Chinese Angelica *(Angelicae Sinensis Radix)* and 20 kg of prepared liauorice root *(Glycyrrhizae Radix et Rhizon Praeparata cum Melle)*. The medicinal materials were weighed according to the formulation ratio, and water of 6 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 21

Medicinal materials were weighed according to the formulation ratio in Examples 16-20, and water of 7 times weight was added, extracted for 3 h, filtrated, and water of 5 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 22

15 kg of Chinese Thorowax Root *(Bupleuri Radix)*, 15 kg of White Peony Root *(Paeoniae Alba Radix)*, 15 kg of Atractylodis Macrocephalae Rhizoma (processed with bran), 15 kg of Chinese Angelica *(Angelicae Sinensis Radix)* and 15 kg of prepared liauorice root *(Glycyrrhizae Radix et Rhizon Praeparata cum Melle)*. The medicinal materials were weighed according to the formulation ratio, and water of 7 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 23

25 kg of Chinese Thorowax Root *(Bupleuri Radix)*, 25 kg of White Peony Root *(Paeoniae Alba Radix)*, 25 kg of Atractylodis Macrocephalae Rhizoma (processed with bran), 25 kg of Chinese Angelica *(Angelicae Sinensis Radix)* and 20 kg of prepared liauorice root *(Glycyrrhizae Radix et Rhizon Praeparata cum Melle)*. The medicinal materials were weighed according to the formulation ratio, and water of 7 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 24

10 kg of Chinese Thorowax Root *(Bupleuri Radix)*, 10 kg of White Peony Root *(Paeoniae Alba Radix)*, 15 kg of Atractylodis Macrocephalae Rhizoma (processed with bran), 10 kg of Chinese Angelica *(Angelicae Sinensis Radix)*, 8 kg of Indian Bread *(Poria)* and 15 kg of prepared liauorice root *(Glycyrrhizae Radix et Rhizon Praeparata cum Melle)*. The medicinal materials were weighed according to the formulation ratio, and water of 6 times weight was added, extracted for 2 h, filtrated, and water of 5 times weight was added into the medicine residue, extracted for 1h, and filtrated. The filtrates from the two extractions were combined, and allowed to stand for 4 h or above under ambient temperature conditions. The supernatant was taken, and the sugar content was controlled at 65%-70% at the concentration endpoint to obtain the traditional Chinese medicine extract.

Example 25

30 kg of Chinese Thorowax Root *(Bupleuri Radix)*, 30 kg of White Peony Root *(Paeoniae Alba Radix)*, 30 kg of Atractylodis Macrocephalae Rhizoma (processed with bran), 30 kg of Chinese Angelica *(Angelicae Sinensis Radix)*, 24 kg of Indian Bread *(Poria)* and 24 kg of prepared liauorice root *(Glycyrrhizae Radix et Rhizon Praeparata cum Melle)*.

The medicinal materials were weighed according to the formulation ratio, and water of 7 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 26

15 kg of Chinese Thorowax Root *(Bupleuri Radix),* 15 kg of White Peony Root *(Paeoniae Alba Radix),* 15 kg of Atractylodis Macrocephalae Rhizoma (processed with bran), 15 kg of Chinese Angelica *(Angelicae Sinensis Radix),* 15 kg of Indian Bread *(Poria)* and 15 kg of prepared liauorice root *(Glycyrrhizae Radix et Rhizon Praeparata cum Melle).* The medicinal materials were weighed according to the formulation ratio, and water of 6 times weight was added, extracted for 2 h, filtrated, and water of 5 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 27

25 kg of Chinese Thorowax Root *(Bupleuri Radix),* 25 kg of White Peony Root *(Paeoniae Alba Radix),* 25 kg of Atractylodis Macrocephalae Rhizoma (processed with bran), 25 kg of Chinese Angelica *(Angelicae Sinensis Radix),* 25 kg of Indian Bread *(Poria)* and 20 kg of prepared liauorice root *(Glycyrrhizae Radix et Rhizon Praeparata cum Melle).* The medicinal materials were weighed according to the formulation ratio, and water of 7 times weight was added, extracted for 3 h, filtrated, and water of 4 times weight was added into the medicine residue, extracted for 1h, and filtrated. The filtrates from the two extractions were combined, and allowed to stand for 4 h or above under ambient temperature conditions. The supernatant was taken, and concentrated to the sugar degree of 65%-70% to obtain the extract.

Example 28

20 kg of Chinese Thorowax Root *(Bupleuri Radix),* 20 kg of White Peony Root *(Paeoniae Alba Radix),* 20 kg of Atractylodis Macrocephalae Rhizoma (processed with bran), 20 kg of Chinese Angelica *(Angelicae Sinensis Radix),* 20 kg of Indian Bread *(Poria)* and 16 kg of prepared liauorice root *(Glycyrrhizae Radix et Rhizon Praeparata cum Melle).* The medicinal materials were weighed according to the formulation ratio, and water of 7 times weight was added, extracted for 2 h, filtrated, and water of 6 times weight was added into the medicine residue, extracted for 1h, and filtrated. The filtrates from the two extractions were combined, and allowed to stand for 4 h or above under ambient temperature conditions. The supernatant was taken, and concentrated to the sugar content of 65%-70% to obtain the extract.

Example 29: Determination of content

The dry content = extract content/dry extract residue, as described in the determination of content below. The dry extract residue is equivalent to solid content, and the detection method refers to the European Pharmacopoeia <2.8.16>.

(1) Determination of saikosaponin B2 content

Chromatography condition: Waters UPLC chromatograph, chromatographic column: BEH C18 (2.1mm*100mm, 1.7 μm) chromatographic column, mobile phase is acetonitrile (A)-water (B), and gradient elution is performed as follows, with the detection wavelength of 254 nm, and the column temperature of 30 °C.

| Time(min) | Flow rate(ml/min) | Mobile phase A (acetonitrile) | Mobile phase B (water) |
|---|---|---|---|
| 0 | 0.3 | 10 | 90 |
| 6 | 0.3 | 38 | 62 |
| 11 | 0.3 | 40 | 60 |
| 14 | 0.3 | 100 | 0 |
| 16 | 0.3 | 100 | 0 |
| 18 | 0.3 | 10 | 90 |

(continued)

| Time(min) | Flow rate(ml/min) | Mobile phase A (acetonitrile) | Mobile phase B (water) |
|---|---|---|---|
| 20 | 0.3 | 10 | 90 |

Preparation of control sample solution:

27.5 mg of saikosaponin B standard sample was precisely weighed and placed in a 50 ml volumetric flask. 15 ml of methanol was added for dissolving the saikosaponin B standard sample. Diluted to volume, and 5 ml was precisely pipetted and placed in a 50 ml volumetric flask. Diluted with methanol to volume, and the solution was mixed evenly to obtain the control sample solution.

Preparation of test sample solution:

0.2 g of the current sample was precisely weighed, and placed in a 50 ml centrifuge tube. 20 ml of 60% methanol -0.5 mol/L NaOH solution was added, subjected to ultrasonic treatment for 40 min, and centrifuged for 10 min. 10 ml of the sample was precisely measured and loaded onto the PSZG column (activated by 10 ml of methanol and 10 ml of purified water in advance), eluted with water until the eluent was neutral, then eluted with 25 ml of methanol and the eluent was collected, and concentrated to dryness under reduced pressure at 50°C, re-dissolved into a 5 ml volumetric flask with methanol, and passed through 0.22 μm organic filter membrane to obtain the test sample solution.
60% methanol -0.5 mol/LNaOH solution: 2 g of sodium hydroxide was taken and dissolved into 100 ml by adding water to obtain 0.5 mol/L NaOH solution. 60 ml of methanol was taken and 0.5 mol/L NaOH solution was added to make it into 100 ml and mixed evenly to obtain 60% methanol -0.5 mol/LNaOH solution. (The amount of various agents and reagents added can be adjusted proportionally according to actual needs).

Determination method

2 μl of the control sample solution and 2 μL of the test sample solution were precisely pipetted and injected into the ultra-performance liquid chromatography for determination, and the dry content of saikosaponin B2 was calculated by external standard method.
Calculation formula:

$$\text{Content (saikosaponin B2)} = \frac{A_{\text{test sample}} \times C_{\text{control sample}} \times 10 \times H}{A_{\text{control sample}} \times m}$$

$$\text{Content (saikosaponin B2)dry weight} = \frac{\text{Content (saikosaponin B2)}}{D}$$

The dry content of saikosaponin B2 is 0.63~10.0mg/g

Note: Content (saikosaponin B2): saikosaponin B2 content, in mg/g;

$A_{\text{test sample}}$:peak area of the test sample;

$C_{\text{control sample}}$: concentration of the control sample solution, in mg/mL;

10: dilution multiples;

H: purity of the control sample;

$A_{\text{control sample}}$: peak area of the control sample;

m: the weighing amount of the test sample, in g;

Content (saikosaponin B2) dry weight: dry content of saikosaponin B2, in mg/g;

D: dry residue, in%.

Methodological validation data:

Injection stability: control sample solution: 36 h; test sample solution: 36 h
Parallel evaluation of sample content results: RAD%≤3.0%

(2) Determination of the content of paeoniflorin

Chromatography conditions:

Waters UPLC chromatograph, chromatographic column: HSS T3 (2.1mm * 100mm, 1.8μm), with acetonitrile as mobile phase A, 0.05% phosphoric acid aqueous solution as mobile phase B, and gradient elution is performed as specified in the following table; column temperature is 30 °C, detection wavelength is 230 nm, and injection volume is 2 μl.
0.05% phosphoric acid aqueous solution: 0.5 ml of phosphoric acid was taken and ultra-pure water was added thereto to make it 1000 ml. (The amount of the reagents added can be adjusted proportionally according to actual needs).

| Time(min) | Flow rate (ml/min) | Mobile phase A (acetonitrile) | Mobile phase B(0.05%phosphoric acid aqueous solution) |
|---|---|---|---|
| 0 | 0.3 | 8 | 92 |
| 16 | 0.3 | 10 | 90 |
| 18 | 0.3 | 100 | 0 |
| 19 | 0.3 | 100 | 0 |
| 21 | 0.3 | 8 | 92 |
| 22 | 0.3 | 8 | 92 |

Preparation of control sample solution:

6 mg of paeoniflorin standard sample was precisely weighed and placed in a 50 ml volumetric flask, and then 15 ml of methanol was added therein for dissolving the paeoniflorin standard sample, the solution was diluted to the scale with purified water, and mixed evenly to obtain the control sample solution.

Preparation of test sample solution:

0.1 g of the current sample was precisely weighed and placed in a 25 ml volumetric flask. An appropriate amount of 30% methanol solution was added, subjected to ultrasonic treatment for 20 min, and dissolved fully. Diluted with 30% methanol solution to volume, and the solution was passed through 0.22 μm organic filter membrane.
30% methanol solution: 30 ml of methanol was taken and water was added thereto to make it 100 ml, and mixed evenly to obtain the methanol solution. (The amount of the reagents added can be adjusted proportionally according to actual needs).

Determination method

2 μl of the control sample solution and 2 μl of the test sample solution were precisely pipetted and injected into the ultra-performance liquid chromatography for determination, and the dry content of paeoniflorin was calculated by external standard method.
Calculation formula:

$$\text{Content (paeoniflorin)} = \frac{A_{\text{test sample}} \times C_{\text{control sample}} \times 25 \times H}{A_{\text{control sample}} \times m}$$

$$\text{Content (paeoniflorin)dry weight} = \frac{\text{Content (paeoniflorin)}}{D}$$

The dry content of paeoniflorin is 9.4~115.9mg/g

Note: Content (paeoniflorin): the dry content of paeoniflorin, in mg/g;

$A_{test\ sample}$: peak area of the test sample;

$C_{control\ sample}$: concentration of the control sample solution, in mg/mL;

25: volume of the test sample solution, in ml;

H: purity of the control sample;

$A_{control\ sample}$: peak area of the control sample;

m: the weighing amount of the test sample, in g;

Content (paeoniflorin) dry weight: dry content of paeoniflorin, in mg/g;

D: dry residue, in%.

Methodological validation data:

Injection stability: control sample solution: 24 h; test sample solution: 24 h

(3) Determination of glycyrrhizic acid content

Chromatography conditions:

Waters UPLC chromatograph, chromatographic column: HSS T3 (2.1mm*100mm, 1.8μm), with acetonitrile as mobile phase A, and 0.05% phosphoric acid aqueous solution as mobile phase B, and gradient elution is performed as specified in the following table; column temperature is 30 °C, and detection wavelength is 230 nm.
0.05% phosphoric acid aqueous solution: 0.5 ml of phosphoric acid was taken and ultra-pure water was added thereto to make it 1000 ml, to obtain the phosphoric acid aqueous solution. (The amount of the reagents added can be adjusted proportionally according to actual needs).

| Time (min) | Flow rate (ml/min) | Mobile phase A (acetonitrile) | Mobile phase B(0.05% phosphoric acid aqueous solution) |
|---|---|---|---|
| 0 | 0.3 | 5 | 95 |
| 5 | 0.4 | 20 | 80 |
| 16 | 0.4 | 55 | 45 |
| 17 | 0.4 | 100 | 0 |
| 18 | 0.4 | 100 | 0 |
| 19 | 0.3 | 5 | 95 |
| 21 | 0.3 | 5 | 95 |

Control sample solution:

6 mg of ammonium glycyrrhizinate standard sample was precisely weighed and placed in a 50 ml volumetric flask, and then 15 ml of methanol was added therein for dissolving the ammonium glycyrrhizinate standard sample, the solution was diluted to the scale with purified water, 5 ml was precisely pipetted, placed in a 25 ml volumetric flask.

Diluted with 30% methanol solution to volume, and the solution was mixed evenly to obtain the control sample solution.

Test sample solution:

0.15 g of the current sample was precisely weighed and placed in a 25 ml volumetric flask. About 15 ml of 30% methanol solution was added, subjected to ultrasonic treatment for 20 min, dissolved fully Diluted with 30% methanol solution to volume, and the solution was passed through 0.22 μm organic filter membrane to obtain the test sample solution.

30% methanol solution: 30 ml of methanol was taken and water was added thereto to make it 100 ml, and mixed evenly to obtain the methanol solution. (The amount of the reagents added can be adjusted proportionally according to actual needs).

Determination method

2 μl of the control sample solution and 2 μl of the test sample solution were precisely pipetted and injected into the ultra-performance liquid chromatography for determination, and the dry content of glycyrrhizic acid was calculated by external standard method.

Calculation formula:

$$\text{Content (glycyrrhizic acid)} = \frac{A_{\text{test sample}} \times C_{\text{control sample}} \times 25 \times H}{A_{\text{control sample}} \times m}$$

$$\text{Content (glycyrrhizic acid)dry weight} = \frac{\text{Content (glycyrrhizic acid)}}{D}$$

The dry content of glycyrrhizic acid is 1.4-26.1 mg/g.

Note: Content (glycyrrhizic acid): dry content of glycyrrhizic acid, in mg/g;

$A_{\text{test sample}}$: peak area of the test sample;

$C_{\text{control sample}}$: concentration of the control sample solution, in mg/ml;

25: volume of the test sample solution, in ml;

H: purity of the control sample;

$A_{\text{control sample}}$: peak area of the control sample;

m: the weighing amount of the test sample, in g;

Content (glycyrrhizic acid) dry weight: dry content of glycyrrhizic acid, in mg/g;

D: dry residue, in%.

Methodological validation data:

Injection stability: control sample solution: 24 h; test sample solution: 24 h

(4) ferulic acid

Chromatography conditions:

Waters UPLC chromatograph, chromatographic column: HSS T3 (2.1mm * 100mm, 1.8μm), with acetonitrile as mobile phase A, and 0.05% phosphoric acid aqueous solution as mobile phase B, and gradient elution is performed as specified in the following table; column temperature is 30 °C, the sample temperature is 20 °C, detection wavelength is 232 nm, and injection volume is 2 μl.

0.05% phosphoric acid aqueous solution: 0.5 ml of phosphoric acid was taken and ultra-pure water was added thereto to make it 1000 ml, to obtain the phosphoric acid aqueous solution. (The amount of the reagents added can be adjusted proportionally according to actual needs.

| Time (min) | Flow rate (ml/min) | Mobile phase A (acetonitrile) | Mobile phase B(0.05% phosphoric acid aqueous solution) |
|---|---|---|---|
| 0 | 0.4 | 5 | 95 |
| 3 | 0.4 | 11 | 89 |
| 12 | 0.4 | 12 | 88 |
| 14 | 0.4 | 20 | 80 |
| 16 | 0.4 | 30 | 70 |
| 17 | 0.4 | 100 | 0 |
| 19 | 0.4 | 5 | 95 |
| 20 | 0.4 | 5 | 95 |

Preparation of control sample solution:

6 mg of ferulic acid standard sample was precisely weighed and placed in a 50 ml volumetric flask, and then 15 ml of methanol was added therein for dissolving the ferulic acid standard sample, the solution was diluted to the scale with purified water, 1 ml was precisely pipetted, placed in a 50 ml volumetric flask, diluted with 30% methanol solution to volume, and the solution was mixed evenly to obtain the control sample solution.

Preparation of test sample solution:

0.15 g of the current sample was precisely weighed and placed in a 25 ml volumetric flask. About 15 ml of 30% methanol solution was added, subjected to ultrasonic treatment for 20 min, dissolved fully, and diluted with 30% methanol solution to volume, and the solution was passed through 0.22 μm organic filter membrane to obtain the test sample solution.

30% methanol solution: 30 ml of methanol was taken and water was added thereto to make it 100 ml, and mixed evenly to obtain the methanol solution. (The amount of the reagents added can be adjusted proportionally according to actual needs).

Determination method

2 μl of the control sample solution and 2 μl of the test sample solution were precisely pipetted and injected into the ultra-performance liquid chromatography for determination, and the dry content of ferulic acid was calculated by external standard method.

Calculation formula:

$$\text{Content (ferulic acid)} = \frac{A_{\text{test sample}} \times C_{\text{control sample}} \times 25 \times H}{A_{\text{control sample}} \times m}$$

$$\text{Content (ferulic acid) dry weight} = \frac{\text{Content (ferulic acid)}}{D}$$

The dry content of ferulic acid is 0.13~0.91mg/g

Note: Content (ferulic acid): dry content of ferulic acid, in mg/g;

$A_{\text{test sample}}$: peak area of the test sample;

$C_{\text{control sample}}$: concentration of the control sample solution, in mg/ml;

25: volume of the test sample solution, in ml;

H: purity of the control sample;

$A_{\text{control sample}}$: peak area of the control sample;

m: the weighing amount of the test sample, in g;

Content (ferulic acid) dry weight: dry content of ferulic acid, in mg/g;

D: dry residue, in%.

Methodological validation data:

Injection stability: control sample solution: 36 h; test sample solution: 36 h

(5) Atractylenolide III

Chromatography conditions:

Waters UPLC chromatograph, chromatographic column: HSS T3 (2.1mm * 100mm, 1.8$\mu$m), with acetonitrile as mobile phase A, and 0.05% phosphoric acid aqueous solution as mobile phase B, and gradient elution is performed as specified in the following table; column temperature is 30 °C, the sample temperature is 20 °C, detection wavelength is 232 nm, and injection volume is 2 $\mu$l.
0.05% phosphoric acid aqueous solution: 0.5 ml of phosphoric acid was taken and ultra-pure water was added thereto to make it 1000 ml, to obtain the phosphoric acid aqueous solution. (The amount of the reagents added can be adjusted proportionally according to actual needs).

| Time (min) | Flow rate (ml/min) | Mobile phase A (acetonitrile) | Mobile phase B(0.05% phosphoric acid aqueous solution) |
|---|---|---|---|
| 0 | 0.3 | 10 | 90 |
| 12 | 0.3 | 12 | 88 |
| 14 | 0.3 | 12 | 88 |
| 22 | 0.3 | 28 | 72 |
| 34 | 0.3 | 45 | 55 |
| 36 | 0.3 | 80 | 20 |
| 39 | 0.3 | 90 | 10 |
| 41 | 0.3 | 90 | 10 |
| 42 | 0.3 | 10 | 90 |
| 44 | 0.3 | 10 | 90 |

Preparation of control sample solution:

6 mg of atractylenolide III standard sample was precisely weighed and placed in a 50 ml volumetric flask, and then 15 ml of methanol was added therein for dissolving the atractylenolide III standard sample, the solution was diluted to the scale with purified water, 5 ml was precisely pipetted, placed in a 50 ml volumetric flask. Diluted with 30% methanol solution to volume, and the solution was mixed evenly to obtain the control sample solution.

Preparation of test sample solution:

0.5 g of the current sample was precisely weighed and placed in a 10 ml volumetric flask. About 7 ml of 30% methanol solution was added, subjected to ultrasonic treatment for 30 min, dissolved fully. Diluted with 30% methanol solution to volume, and the solution was passed through 0.22 $\mu$m organic filter membrane to obtain the test sample solution.

30% methanol solution: 30 ml of methanol was taken and water was added thereto to make it 100 ml, and mixed evenly to obtain the methanol solution. (The amount of the reagents added can be adjusted proportionally according to actual needs).

Determination method

2 $\mu$l of the control sample solution and 2 $\mu$l of the test sample solution were precisely pipetted and injected into the ultra-performance liquid chromatography for determination, and the dry content of atractylenolide III was calculated by external standard method.

Calculation formula:

$$\text{Content (atractylenolide III)} = \frac{A_{\text{test sample}} \times C_{\text{control sample}} \times 10 \times H}{A_{\text{control sample}} \times m}$$

$$\text{Content (atractylenolide III)dry weight} = \frac{\text{Content (atractylenolide III)}}{D}$$

The dry content of atractylenolide III is 0.0038~0.30mg/g.

Note: Content (atractylenolide III): dry content of atractylenolide III, in mg/g;

$A_{\text{test sample}}$: peak area of the test sample;

$C_{\text{control sample}}$: concentration of control sample solution, in mg/ml;

10: volume of the test sample solution, in ml;

H: purity of the control sample;

$A_{\text{control sample}}$: peak area of the control sample;

m: the weighing amount of the test sample, in g;

Content (Atractylenolide III) dry weight: dry content of atractylenolide III, in mg/g;

D: dry residue, in%.

Methodological validation data:

Injection stability: control sample solution: 48 h; test sample solution: 48 h

Parallel evaluation of sample content results: RAD%$\leq$8.5%

Example 30. antidepressant effect experiment

Test example I

Chronic unpredictable mild stress (CUMS) combined with solitary depression rat model is the closest animal model to clinical depression. It means that rats are exposed to continuous mild and unpredictable stimulation conditions during the modeling process, and combined with solitary depression to induce rats to produce many behavioral abnormalities similar to those of patients with depression. Among them, the body weight change, the percentage of sugar water consumption and the score of open-box exercise were the main behavioral evaluation indices of the depression model, which can reflect the symptoms, such as poor appetite, loss of pleasant sensation, self-activity ability, and reduced curiosity about the fresh environment, of patients with clinical depression.

The pathogenesis of depression is complex. At present, widely accepted and deeply studied hypotheses include monoamine neurotransmitter hypothesis, hypothalamic-pituitary-adrenal (HPA) axis hyperthyroidism hypothesis and neuroplasticity hypothesis. Among them, the monoamine neurotransmitter hypothesis, as the most classic hypothesis of the depression, holds that the occurrence of the depression is mainly due to the lack of monoamine neurotransmitters such as 5- hydroxytryptamine (5-HT), dopamine (DA) and norepinephrine (NE) in the central nervous system of the brain, which is also the action target of most antidepressants clinically at present. The hypothalamic-pituitary-adrenal (HPA) axis hyperthyroidism can lead to abnormal increase of corticosterone (CORT) content in the body, which leads to neurotoxicity after binding with its receptor, resulting in damage of neurons in the hippocampus, thus causing the depression. The neuroplasticity hypothesis holds that brain-derived neurotrophic factor (BDNF) is involved in the regulation of the survival, growth, differentiation and apoptosis of nerve cells, and it can resist the damage of neurons under stress state, promote the repair and regeneration of neurons, and improve the plasticity of neurons, thus playing an antidepressant role. Therefore, in this experiment, CUMS combined with solitary depression rat model was used, and the behavioral and biochemical indices (CORT, DA, NE, 5-HT, and BDNF) content of CUMS combined with solitary depression rat model was taken as the pharmacodynamics index of antidepressant effect. The pharmacodynamics of antidepressant effects of *Jiawei Xiaoyao Wan* before and after the prescription was reduced were compared.

1 Experimental animals and materials

1.1 Experimental animals

130 SPF male Sprague Dawley(SD) rats [purchased from China Institute of Food and Drug Control, license number: SCXK (Beijing) 2017-0005], weighing 180 ~ 200 g, were adaptively raised and tested in the Experimental Animal Center of Tianjin University of Traditional Chinese Medicine. Feeding conditions: $(22\pm2)$ °C, humidity $(50\pm10)$%, natural light, free access to food and water. The feeding and operating rules of experimental animals comply with the relevant regulations of Tianjin University of Traditional Chinese Medicine on the feeding and use of experimental animals.

1.2 Drugs and reagents

The extracts of A group (*Jiawei Xiaoyao Fang*), C group (*Xiaoyao Fang*), D group (Example 16 of the present application), E group (Example 28 of the present application) and F group (prepared with reference to Chinese patent CN108653405A) were prepared according to the formulations provided by Tasly Pharmaceutical Group Co., Ltd. in Table 1; fluoxetine hydrochloride (Flu) was produced in Lilly Suzhou Pharmaceutical Co., Ltd.; plasma corticosterone (CORT) ELISA kit, neurotrophic factor (BDNF) ELISA kit, 5-hydroxy tryptamine (5- HT) ELISA kit, dopamine (DA) ELISA kit and norepinephrine (NE) ELISA kit were all purchased from R&D Systems in the United States; normal saline was purchased from Shijiazhuang No.4 Pharmaceutical Co., Ltd..

1.3 Experimental equipment and instruments

Open box (100 cm×100 cm×50 cm, self-made), LTD0114 bullet warning lamp (Zhejiang Anhua Safety Equipment Co., Ltd.), G6805-1 therapeutic instrument (Qingdao Xinsheng Industrial Co., Ltd., China), wooden clip, TD21001 electronic balance (Tianjin Tianma Instrument Factory), IKA T18 ultrasonic tissue breaker (Ningbo Xinzhi Biotechnology Co., Ltd.), 3K15 model frozen high-speed centrifuge (Sigma Company, USA), Infinite M200 Pro type multifunctional microplate reader (TECAN Company, Switzerland), -80 °C low-temperature refrigerator (Thermo Technology Company, USA).

2 Experimental methods

2.1 Preparation of drug solution

2.1.1 Preparation of fluoxetine suspension

Fluoxetine hydrochloride capsules (20 mg/ capsule) were taken and the shell of the capsule was removed, and 0.5% CMC-Na solution was added therein, grinded and formulated into 1 mg/mL of fluoxetine suspension.

2.1.2 Preparation of drug solution in each group

The daily clinical dosage of the extracts from A group, C group, D group, E group and F group were 5.21 g, 3.21 g, 3.26 g, 3.29 g and 2.55 g, respectively, and the doses for the rat converted according to 15 times the clinically equivalent dose were 7.0305 g/kg, 4.3380 g/kg, 4.4040 g/kg, 4.4415 g/kg and 3.4425 g/kg, respectively, as shown in Table 1.

Table 1 Conversion table of intragastric dose for rats

| Group | Medicinal material | Feeding amount (kg) | Paste rate | Amount of extract used (g/day) | Amount of raw drug (g/day) | 15 times the amount of extract used (g/day) | Dose in rats (g/kg) |
|---|---|---|---|---|---|---|---|
| The medicine of A group *JiaWeiXiao Yao* ten medicinal materials were combined and extracted with water | Chinese Thoro wax Root *(Bupleuri Radix)* | 0.6 | 43.39% | 12×43.39%= 5.21 | 12 | 5.21×15= 78.15 | 7.0335 |
| | White Peony Root *(Paeoniae Alba Radix)* | 0.6 | | | | | |
| | Chinese Angelica *(Angelicae Sinensis Radix)* | 0.6 | | | | | |
| | Atractiodis Macro cephalae Rhizoma (processed with bran) | 0.6 | | | | | |
| | prepared liauorice root *(Glycyrrhizae Radix et Rhizon Praeparata cum Melle)* | 0.48 | | | | | |
| | Fructus Gardeniae | 0.9 | | | | | the dose for the rat converted according to 15 times the clinically equivalent dose is 7.0335g/kg |
| | Moutan Cortex | 0.9 | | | | | |
| | Pepper mint | 0.12 | | | | | |
| | Dried ginger | 0.2 | | | | | |
| | Indian Bread *(Poria)* | 0.6 | | | | | |

(continued)

| Group | Medicinal material | Feeding amount (kg) | Paste rate | Amount of extract used (g/day) | Amount of raw drug (g/day) | 15 times the amount of extract used (g/day) | Dose in rats (g/kg) |
|---|---|---|---|---|---|---|---|
| The medicine of C group *(Xiaoyao formulation )* seven medicine materials | Chinese Thoro wax Root *(Bupleuri Radix)* | 0.9 | 42.81 % | 7.5×42.81 %= 3.21 | 7.5 | 3.21×15= 48.15 | 4.3380 |
| were combined and extracted with water | White | 0.9 | | | | | |
| | Peony Root *(Paeoniae Alba Radix)* | | | | | | |
| | Chinese Angelica *(Angelicae Sinensis Radix)* | 0.9 | | | | | |
| | Atractylodis Macrocephalae Rhizoma (processed with bran) | 0.9 | | | | | |
| | prepared liauorice root *(Glycyrrhizae Radix et Rhizon Praepa rata cum Melle)* | 0.72 | | | | | |
| | Pepper mint | 0.18 | | | | | |
| | Indian Bread *(Poria)* | 0.9 | | | | | |
| The medicine of D group (Example | Chinese Thoro wax | 1 | 54.38 % | 3.26 | 6 | 48.90 | 4.4040 |

(continued)

| Group | Medicinal materia l | Feeding amount (kg) | Paste rate | Amount of extract used (g/day) | Amount of raw drug (g/day) | 15 times the amount of extract used (g/day) | Dose in rats (g/kg) |
|---|---|---|---|---|---|---|---|
| 16) five medicinal materials were combined and extracted with water , and Indian Bread (Poria) and Peppermint were removed from Xiaoyao formulation | Root (Bupleuri Radix) | | | | | | |
| | White Peony Root (Paeoniae Alba Radix) | 1 | | | | | |
| | Chinese Angeli ca (Angeliae Sinensi s Radix) | 1 | | | | | |
| | Atracty lodis Macro cephal ae Rhizo ma (proces sed with bran) | 1 | | | | | |
| | prepare d liauori ce root (Glycy rrhizae Radix et Rhizon Praepa rata cum Melle) | 0.8 | | | | | |
| The medicine of E group (Example 28) six | Chinese Thoro wax Root | 1 | 45.34 % | 3.29 | 7.25 | 49.35 | 4.4415 |

(continued)

| Group | Medici nal materia l | Feedi ng amou nt (kg) | Paste rate | Amount of extract used (g/ day) | Amou nt of raw drug (g/ day) | 15 times the amount of extract used (g/ day) | Dose in rats (g/kg) |
|---|---|---|---|---|---|---|---|
| medicinal materials were combined and extracted with water , and Peppermint was removed from the Xiaoyao formulation | *(Buple uri Radix)* | | | | | | |
| | White Peony Root *(Paeon iae Alba Radix)* | 1 | | | | | |
| | Chines e Angeli ca *(Angeli cae Sinensi s Radix)* | 1 | | | | | |
| | Atracty lodis Macro cephal ae Rhizo ma (proces sed with bran) | 1 | | | | | |
| | prepare d liauori ce root *(Glycy rrhizae Radix et Rhizon Praepa rata cum Melle)* | 0.8 | | | | | |
| | Indian Bread *(Poria)* | 1 | | | | | |
| The medicine of | Chines e | 0.84 | 42.44 % | 2.55 | 6 | 38.25 | 3.4425 |

(continued)

| Group | Medicinal materia l | Feeding amou nt (kg) | Paste rate | Amount of extract used (g/ day) | Amou nt of raw drug (g/ day) | 15 times the amount of extract used (g/ day) | Dose in rats (g/kg) |
|---|---|---|---|---|---|---|---|
| F group Six medicinal materials were combined and prepared according to the extraction process described in Chinese patent CN108653 405A | Thoro wax Root (Buple uri Radix) | | | | | | |
| | White Peony Root (Paeon iae Alba Radix) | 0.84 | | | | | |
| | Chines e Angeli ca (Angeli cae Sinensi s Radix) | 0.84 | | | | | |
| | Atracty lodis Macro cephal ae Rhizo ma (proces sed with bran) | 0.84 | | | | | |
| | prepare d liauori ce root (Glycy rrhizae Radix et Rhizon Praepa rata cum Melle) | 0.42 | | | | | |
| | Pepper mint | 0.28 | | | | | |

$$\text{Paste rate } (\%) = \text{weight of the extract / weight of the medicinal material} \times 100\%$$

The extraction method of the A group, B group and C group medicines:

All the medicinal materials were weighted according to their respective formulation ratio, water of 6 times weight was added, extracted for 2 hours, and filtered. Water of 5 times weight was added into the medicine residue, extracted for 1 h, and filtered. The filtrates from the two extractions were combined, and allowed to stand for 4 h or above under ambient temperature conditions. The supernatant was taken, and controlled to 65%-70% sugar content at the concentration endpoint to obtain the traditional Chinese medicine extract.

2.2 Establishment of CUMS combined with solitary depression model

After 130 SD rats were raised adaptively for 7 days, the open-box experiment was carried out in a quiet environment. The rats were screened according to the principle that the scores of horizontal movement and vertical movement were similar, and the rats with too high or too low scores were excluded. Screened rats were randomly divided into normal group, model group, Flu group, A group, C group, D group, E group, and F group, with 13 rats in each group.
The rats in the normal group were raised normally with four rats per cage, with free access to water and food, and without receiving any other additional stimulation. Rats in other groups were raised in a single cage, and received the following two different stress stimuli randomly every day, and the same stimuli cannot appear continuously, so that the rats cannot predict what kind of stimuli and avoid their adaptability. CUMS stimulation factors include: restraint for 2 h, ice water platform for 2 h, noise stimulation (110 dB, 30 min), plantar electric shock (current 4 mA, 50 times, with an interval of 5 s each time), light stimulation (100 000 lx, 30 min), inversion of day and night for 24 h, wet bedding for 12 h, and tail clamping (about 1 cm from the tail tip of rats, 2 min). Rats in each group were continuously modeled, and their body weight gain, the percentage of sugar water consumption, and the scores of horizontal and vertical movements in the open box experiments were measured every week.

2.3 Measurement of pharmacodynamic indices

2.3.1 Behavioral experiments

(1) Body weight change

The body weight of the rats was measured on days 0, 7, 14, 21, 28, 35, 42, 49, 56, and 63 of the experiment, respectively. The body weight of the rats in each group was recorded, and the results of weekly body weight changes of the rats in each group during the modeling process and the body weight gain (%) before and after modeling were analyzed. The formula is as follows:

$$\text{Weight gain}(\%) = \frac{(\text{Weight on day } 63 - \text{Weight on day } 0)}{\text{Weight on day } 0} \times 100\%$$

(2) Sugar water consumption experiments

The sugar water consumption experiments were conducted on rats in each group on days 0, 7, 14, 21, 28, 35, 42, 49, 56 and 63 of the experiment. Three days before the experiment, sugar water adaptation was carried out. Two bottles of 1% sucrose solution were placed on the cage for 24 h, followed by one bottle of 1% sucrose solution and one bottle of pure water for 24 h. The positions of the water bottles were changed in the morning and afternoon, and water and food were forbidden for 24 h on day 3. In the formal experiment, one bottle of 1% sucrose solution and one bottle of pure water were placed for the rats in each group. The positions of the water bottles were changed in the morning and afternoon. The amount of sucrose solution (the amount of sugar water consumption) and the amount of pure water consumption within 24 h in each group were recorded, and the percentage of sugar water consumption (%) was calculated. The formula is as follows:

$$\text{the percentage of sugar water consumption}(\%) =$$

$$\frac{\text{the amount of sugar water consumption}}{(\text{the amount of sugar water consumption} + \text{the amount of pure water consumption})} \times 100\%$$

(3) Open-box experiment

The rats in each group were subjected to open-box experiment on days 0, 7, 14, 21, 28, 35, 42, 49, 56 and 63 of the experiment. The size of the open box used in the experiment is 100 cm×100 cm×50 cm (length× width× height), and the inner side is black. The bottom surface is divided into 100(10 cm×10 cm) small squares of the same size with white lines. Each rat was placed in a fixed square in the center in turn. The activity route of rats within 3 minutes without human interference was recorded. The number of the experimental rats crossing the small squares on the bottom surface was taken as the score of horizontal movement. Every time the rat crosses one square (entering with more than 3 claws), it was counted as one point for horizontal movement. If the experimental rats walked along a straight line, it was counted as one point for every 10 cm. The number of times the experimental rats stood upright was taken as the score of vertical movement, and the separation of both forelimbs from the bottom surface of the open box is the mark of vertical movement. No matter how long the rats stood, the laying down of both forelimbs was taken as the vertical movement score of 1 point. After each rat was tested, the open box was thoroughly cleaned and then the next rat was tested. The scores of horizontal and vertical movements of each rat were recorded, and the differences in the scores of horizontal and vertical movements of each group were analyzed.

2.3.2 Detection of biochemical indices

(1) Acquisition of materials

After the behavioral experiment, about 1.5 mL of blood was taken from the rat's orbit, and placed in a centrifuge tube soaked with heparin at 8000 r·min$^{-1}$ at 4 °C for 10 min. The supernatant was taken and placed in a refrigerator at -80 °C for storage, for the determination of corticosterone (CORT) level in the plasma of rat.

After blood was taken from the orbit, the neck was removed and killed. The cortex and hippocampus were quickly peeled off on the ice surface, weighed, placed in a freezing tube and quickly frozen in liquid nitrogen, and then transferred to a refrigerator at -80 °C for storage. The contents of nerve growth nutrition factor (BDNF), 5-hydroxytryptamine (5-HT), dopamine (DA) and norepinephrine (NE) in the cortex and hippocampus of rats were determined.

2.3.3.2 Determination and calculation of CORT, BDNF, 5-HT, DA and NE

Enzyme linked immunosorbent assay (ELISA) was used to determine the content of CORT in the plasma, and the contents of BDNF, 5-HT, DA and NE in the cortex and hippocampus of rats in each group, and the absorbance (OD value) of each well was measured in sequence at 450 nm wavelength. A standard curve was plotted with the content of standard substance as abscissa and OD value as ordinate, and obtained a regression equation. The OD values of CORT in the plasma and BDNF, 5-HT, DA and NE in the cortex and hippocampus of rats in each group are substituted into the regression equation, and the contents of CORT in the plasma and BDNF, 5-HT, DA and NE in the cortex and hippocampus of rats in each group are calculated.

2.4. Experimental data processing and statistical analysis

The experimental data were expressed as $\bar{x} \pm s$ and were analyzed by SPSS 22.0 statistical software. The content of CORT in the plasma and the contents of BDNF, 5-HT, DA and NE in the cortex and hippocampus of rats were analyzed for inter-group difference analysis by One-way ANOVA.

3 Experimental results and analysis

3.1 Weight change results

Different degrees of body weight gain were observed for rats in each experimental group as the modeling process proceeds, and the results are shown in Table 2. The body weight gain of the rats in the model group was decreased significantly as compared with that in the normal group (P<0.01), which indicated that CUMS combined with the solitary modeling method affected the appetite of rats, resulted in slow body weight gain, and simulated the symptoms of anorexia and weight loss in the patients with depression.

The body weight gain of rats in each administration group was significantly higher than that in the model group (P<0.01), which indicated that each administration group can resist the body weight loss of rats with CUMS combined with solitary depression model, and thus improve the symptoms of anorexia and weight loss of rats with CUMS combined with solitary depression model. From the table, it can be seen that, on the whole, the relationships between the body weight gains of rats in each administration group are in the order of Flu group > D group > F group > E group > C group >A group, which indicates that the effect of extract in D group on improving the symptoms of anorexia and weight loss of rats with CUMS combined with solitary depression is second only to Flu group medicine, and the effect of extract in A group on improving the symptoms of anorexia and weight loss of rats with CUMS combined with solitary depression is the worst. There were significant differences between D group and E group (P<0.01).

Table 2 Comparison of weight gain of rats in each group ($\bar{x} \pm s$, n=10)

| Group | Dosage(g/kg) | Weight on day 0(g) | Weight on day 63(g) | Weight gain(%) |
|---|---|---|---|---|
| Normal group | - | 226.00±10.86 | 458.50±21.12 | 102.95±5.47 |
| Model group | - | 219.12±9.37 | 308.00±13.45 ▼ | 40.56±1.69▼ |
| A group | 7.03 | 219.62±7.55 | 334.00±18.01# | 52.11±7.24# |
| C group | 4.34 | 222.00±8.00 | 338.87±12.47# | 52.87±8.85# |
| D group | 4.40 | 223.00±4.31 | 423.37±20.43# | 89.82±7.43# |

(continued)

| Group | Dosage(g/kg) | Weight on day 0(g) | Weight on day 63(g) | Weight gain(%) |
|---|---|---|---|---|
| E group | 4.44 | 222.63±10.98 | 381.25±13.19# | 71.65±10.95# |
| F group | 3.44 | 221.75±4.77 | 383.75±10.67# | 73.11±5.56# |
| Flu group | 0.01 | 226.25±9.13 | 426.88±8.92# | 89.02±10.31# |
| Compared with the normal group, ▽P<0.05, ▼ P < 0.01; compared with the model group, * p < 0.05, # P<0.01 | | | | |

3.2 Results of behavioral experiments

3.2.1 Results of the percentage of sugar water consumption

The percentage of sugar water consumption of rats in each experimental group decreased gradually with the modeling process, and the results are shown in Table 3. The percentage of sugar water consumption in the model group decreased significantly as compared with that in the normal group (P<0.01), which indicated that CUMS combined with solitary modeling method reduced the percentage of sugar water consumption in rats, and simulated the symptoms of anhedonia in the patients with depression.

The percentage of sugar water consumption in each administration group was significantly higher than that in the model group (P<0.01), which indicated that the symptoms of anhedonia of rats with CUMS combined with solitary depression was improved in each administration group. It can be seen from the table that, on the whole, the relationships of the percentages of sugar water consumption of rats in each administration group were in the order of Flu group > D group > E group > F group > C group > A group, which indicates that the effect of the extract in D group on improving the symptoms of anhedonia of rats with CUMS combined with solitary depression is second only to that of the extract in Flu group medicine, and that the effect of the extract in A group on improving the symptoms of anhedonia of rats with CUMS combined with solitary depression is the worst. There were significant differences between D group and E group (P<0.01).

Table 3 Results of the percentage of sugar water consumption of rats in each group ($\bar{x}±s$, n=10)

| Group | Dosage(g/kg) | On day 0(%) | On day 63(%) |
|---|---|---|---|
| Normal group | - | 89.41±3.47 | 93.17±3.80 |
| Model group | - | 90.11±2.63 | 61.75±3.63▼ |
| A group | 7.03 | 89.53±4.21 | 67.92±3.60# |
| C group | 4.34 | 89.72±5.77 | 68.63±4.38# |
| D group | 4.40 | 90.69±2.33 | 88.31±4.14# |
| E group | 4.44 | 90.81±3.52 | 79.12±2.74# |
| F group | 3.44 | 90.28±2.86 | 78.67±4.51# |
| Flu Group | 0.01 | 89.99±3.77 | 88.62±2.97# |
| Compared with the normal group, ▽P<0.05, ▼ P < 0.01; compared with the model group, * p < 0.05, # P<0.01 | | | |

3.2.2 Results of the open-box experiment

(1) Score of horizontal movement

The scores of open-box horizontal movement of rats in each experimental group decreased gradually with the modeling process, and the results of the scores of open-box horizontal movement of rats in each group are shown in Table 4. The score of open-box horizontal movement of rats in the model group decreased significantly as compared with that in the normal group (P<0.01), which indicated that CUMS combined with solitary modeling method can significantly reduce the emotion and autonomous activity of rats, and simulate the symptoms of autonomous hypoactivity in the patients with depression.

The scores of open-box horizontal movement in each administration group were significantly higher than those in the model group (P<0.01), which indicated that each administration group can effectively up-regulate the emotion and autonomous activity of rats with CUMS combined with solitary depression model, and thus improve the symptoms of autonomous hypoactivity of rats with CUMS combined with solitary depression model. It can be seen from the table that, on the whole, the relationships of the scores of horizontal movement of rats in each administration group were in the order of Flu group > D group > E group > F group > C group >A group, which indicates that the effect of the extract in D group on improving the symptoms of hypoactivity of rats with CUMS combined with solitary depression model is second only to Flu group medicine, and the effect of the extract in A group on improving the symptoms of hypoactivity of rats with CUMS combined with solitary depression model is the worst. There were significant differences between D group and E group (P<0.01).

Table 4 Results of the scores of horizontal movement of rats in each group in open-box experiment ($\overline{x}\pm s$, n=10)

| Group | Dosage(g/kg) | On day 0(%) | On day 63(%) |
|---|---|---|---|
| Normal group | - | 243.25±38.10 | 178.63±19.27 |
| Model group | - | 226.62±25.72 | 71.37±12.11▼ |
| A group | 7.03 | 245.75±51.12 | 88.13±12.28 * |
| C group | 4.34 | 211.25±46.64 | 89.50±15.27 * |
| D group | 4.40 | 229.13±62.84 | 162.75±30.07# |
| E group | 4.44 | 205.00±30.39 | 127.75±23.57# |
| F group | 3.44 | 233.25±38.54 | 119.38±24.54# |
| Flu group | 0.01 | 239.50±35.31 | 173.25±11.94# |
| Compared with the normal group, ∇P<0.05, ▼P < 0.01; Compared with the model group, * p < 0.05, # P<0.01 | | | |

(2) Score of vertical movement

The scores of vertical and horizontal movements of rats in each experimental group decreased gradually with the modeling process, and the results of the scores of open-box vertical movements of rats in each group are shown in Table 5. The score of open-box vertical movement of rats in the model group was decreased significantly as compared with that in the normal group (P<0.01), which indicated that CUMS combined with solitary modeling method can significantly reduce the spatial exploration ability of rats, and simulate the symptoms of reduced curiosity of the patients with depression about the fresh environment.

The scores of open-box vertical movement in each administration group were significantly higher than those in the normal group (P<0.05), which indicated that the symptoms of reduced curiosity of rats with CUMS combined with solitary depression model about fresh environment can be improved in each administration group. From the table, it can be seen that, on the whole, the relationships of the scores of vertical movements of rats in each administration group were in the order of Flu group > D group > E group > F group > C group > A group, which indicates that the effect of the extract in D group on improving the symptoms of reduced curiosity of rats with CUMS combined with solitary depression model about the fresh environment is second only to Flu group medicine, and the effect of the extract in A group on improving the symptoms of reduced curiosity of rats with CUMS combined with solitary depression model about the fresh environment is the worst. There were significant differences between D group and E group (P<0.05).

Table 5 Results of the scores of vertical movements of rats in each group in open-box experiment ($\overline{x}\pm s$, n=10)

| Group | Dosage(g/kg) | On day 0 | On day 63 |
|---|---|---|---|
| Normal group | - | 11.50±2.07 | 9.38±1.92 |
| Model group | - | 12.75±2.12 | 2.38±1.41▼ |
| A group | 7.03 | 13.00±3.21 | 4.00±0.76 * |
| C group | 4.34 | 11.25±2.82 | 4.25±1.39 * |
| D group | 4.40 | 10.63±3.54 | 7.25±2.19# |
| E group | 4.44 | 10.57±4.28 | 6.13±2.10# |
| F group | 3.44 | 12.75±1.67 | 6.00±2.45# |

(continued)

| Group | Dosage(g/kg) | On day 0 | On day 63 |
|---|---|---|---|
| Flu group | 0.01 | 12.00±1.51 | 8.75±1.83# |

Compared with the normal group, ∇P<0.05, VP < 0.01; compared with the model group, *p < 0.05, # P<0.01

3.3.3 Test results of biochemical indicators

(1) Comparison of the contents of CORT in the plasma of rats in each group

The contents of CORT in the plasma of rats in each group are shown in Table 6. The contents of CORT in the plasma of rats in the model group increased significantly as compared with those in the normal group (P<0.01), which indicated that CUMS combined with solitary modeling method significantly increased the contents of CORT in the plasma of rats, resulting in HPA axis hyperfunction and thus depression in rats.

The contents of CORT in the plasma of rats in each administration group were decreased significantly as compared with those in the model group (P<0.01), which indicated that the HPA axis hyperfunction of rats with depression with CUMS combined with solitary model can be improved in each administration group, and thus an antidepressant role can be exerted. It can be seen from the table that, on the whole, the relationships of the contents CORT in the plasma of rats in each administration group were in the order of Flu group > D group > F group > E group > C group > A group, which indicated that the antidepressant effect of the extract in D group is second only to Flu group medicine, and the antidepressant effect of the extract in A group is the worst. There were significant differences between D group and E group (P<0.01).

Table 6 Comparison of the contents of CORT in the plasma of rats in each group ($\overline{x}±s$, n=10)

| Group | Dosage(g/kg) | CORT(ng/L) |
|---|---|---|
| Normal group | - | 307.7622±8.4732 |
| Model group | - | 431.2767±8.0006▼ |
| A group | 7.03 | 405.5870±1.3391# |
| C group | 4.34 | 390.3106±3.2073# |
| D group | 4.40 | 327.1428±2.1968# |
| E group | 4.44 | 331.6605±2.2757# |
| F group | 3.44 | 338.0807±0.3857# |
| Flu group | 0.01 | 317.8717±7.6536# |

Compared with the normal group, ∇P<0.05, VP < 0.01; compared with the model group, * p < 0.05, # P<0.01

(2) Comparison of the contents of DA, NE, 5-HT and BDNF in the cortex of rats in each group

The contents of DA, NE, 5-HT and BDNF in the cortex of rats in each group were shown in Table 7. The contents of DA, NE, 5-HT and BDNF in the cortex of rats in the model group were significantly decreased as compared with those of the normal group (P<0.01), which indicated that CUMS combined with the solitary modeling method significantly reduced the contents of monoamine neurotransmitters DA, NE, 5-HT and the contents of brain-derived neurotrophic factor BDNF in the cortex of rats, resulting in depression in rats.

The contents of DA, NE, 5-HT and BDNF in the cortex of rats in each administration group were increased significantly as compared with those in the model group (P<0.01), which indicated that in each administration group, the level of monoamine neurotransmitters in the cortex of rats with CUMS combined with solitary depression can be improved, and the repair and regeneration of neurons were promote by up-regulating the BDNF content in the cortex, and thus an antidepressant role was exerted. It can be seen from the table that, on the whole, the relationships of the contents of DA, NE, 5-HT in the cortex of rats in each administration group were in the order of Flu group > D group > F group > E group > C group > A group, and the content of BDNF in the cortex of rats in each administration group was in the order of D group > Flu group > F group > E group > C group > A group, which indicates that the extract in D group has the best antidepressant effect, and the extract in A group has the worst antidepressant effect. There were significant differences in DA content, NE content and 5-HT content in the cortex between D group and E group (P<0.05). The BDNF content in D group was significantly different from that in E group (P<0.01).

Table 7 Comparison of the contents of DA, NE, 5-HT and BDNF in the cortex of rats in each group ($\overline{x}\pm s$ , n=10)

| Group | Dosage(g/kg) | DA(ng/g) | NE(ng/g) | 5-HT(ng/g) | BDNF(ng/g) |
|---|---|---|---|---|---|
| Normal group | - | 0.3278±0.0056 | 0.6569±0.0124 | 1.3358±0.0057 | 2.6500±0.0119 |
| Model group | - | 0.3157±0.0014▼ | 0.4949±0.0208▼ | 1.0881±0.0012▼ | 1.8953±0.0986▼ |
| A group | 7.03 | 0.3177±0.0004# | 0.5311±0.0277* | 1.1052±0.0055# | 2.0695±0.0223# |
| C group | 4.34 | 0.3177±0.0015# | 0.5344±0.0177# | 1.1330±0.0033# | 2.1522±0.0089# |
| D group | 4.40 | 0.3276±0.0049# | 0.6202±0.0144# | 1.1969±0.0038# | 2.5719±0.0111# |
| E group | 4.44 | 0.3227±0.0018# | 0.5977±0.0169# | 1.1893±0.0068# | 2.4664±0.0054# |
| F group | 3.44 | 0.3250±0.0053# | 0.5777±0.0115# | 1.1780±0.00754# | 2.4734±0.0065# |
| Flu group | 0.01 | 0.3279±0.0023# | 0.6343±0.0218# | 1.2104±0.0027# | 2.5390±0.0207# |
| Compared with the normal group, ∇P<0.05, ▼P < 0.01; compared with the model group, * p < 0.05, # P<0.01. | | | | | |

(3) Comparison of the contents of DA, NE, 5-HT and BDNF in the hippocampus of rats in each group

The Comparison of the contents of DA, NE, 5-HT and BDNF in the hippocampus of rats in each group were shown in Table 8. The contents of DA, NE, 5-HT and BDNF in the hippocampus of the model group were significantly decreased as Compared with those in the normal group (P<0.01), which indicated that CUMS combined with the solitary modeling method significantly reduced the contents of monoamine neurotransmitters DA, NE, 5-HT and the content of the brain-derived neurotrophic factor BDNF in the hippocampus of the rats, resulting in the depression of the rats.
The contents of DA, NE, 5-HT and BDNF in the hippocampus of rats in each administration group were significantly increased as compared with those in the model group(P<0.01), which indicated that in each administration group, the level of monoamine neurotransmitters in the hippocampus of rats with CUMS combined with solitary depression model can be improved, and the repair and regeneration of neurons were promoted by up-regulating BDNF content in the hippocampus, and thus an antidepressant role was exerted. It can be seen from the table that, on the whole, the relationships of the contents of DA, NE, 5-HT in the hippocampus of rats in each administration group were in the order of Flu group > D group > F group > E group > C group > A group, and the content of BDNF in the hippocampus of rats in each administration group was in the order of D group > Flu group > F group > E group > C group > A group, which indicates that the extract in D group has the best antidepressant effect, and the extract in A group has the worst antidepressant effect. The contents of DA and NE in the hippocampus of D group were significantly different from those of E group (P < 0.05). The 5-HT content and BDNF content in D group were significantly different from those in E group (P<0.01).

Table 8 Comparison of the contents of DA, NE, 5-HT and BDNF in the hippocampus of rats in each group (x±s, n=10

| Group | Dosage(g/kg ) | DA(ng/g) | NE(ng/g) | 5-HT(ng/g) | BDNF(ng/g) |
|---|---|---|---|---|---|
| Norma I group | - | 0.4530±0.0020 | 0.7797±0.0254 | 1.7650±0.0637 | 4.9568±0.0331 |
| Model group | - | 0.3536±0.0019 ▼ | 0.5851±0.0267 ▼ | 1.0174±0.1016 ▼ | 2.080±0.0151 ▼ |
| A group | 7.03 | 0.3789±0.0020# | 0.6367±0.0187# | 1.1823±0.0254# | 2.6912±0.4591 # |
| C group | 4.34 | 0.3818±0.0013# | 0.6427±0.0308# | 1.2586±0.0223# | 2.7648±0.0245 # |
| D group | 4.40 | 0.4374±0.0040# | 0.7432±0.0222# | 1.5255±0.0216# | 4.7792±0.0263 # |
| E group | 4.44 | 0.4280±0.0015# | 0.7119±0.0322# | 1.4571±0.0154# | 3.7228±0.0246 # |
| F group | 3.44 | 0.4268±0.0023# | 0.7080±0.0217# | 1.4004±0.0344# | 4.0220±0.0379 # |
| Flu group | 0.01 | 0.4466±0.0015# | 0.7569±0.0376# | 1.602±0.0300# | 4.7083±0.0424 # |
| Compared with the normal group, ∇P<0.05, ▼P < 0.01; compared with the model group, * p < 0.05, # P<0.01. | | | | | |

4. Summary

The depression model of rat was established by chronic unpredictable mild stress (CUMS) combined with solitary method. CUMS can significantly reduce the body weight gain in the rat, the percentage of sugar water consumption, and the scores of the horizontal and vertical movements in the open box experiment, and successfully simulate the symptoms of weight loss, lack of pleasure, and reduced self-activity ability and curiosity about the fresh environment in clinical patients with depression, which indicates that CUMS combined with solitary depression model of rats was successfully established.

The results of the order of pharmacodynamic index results of rats in each group are shown in Table 9. It can be seen from the table that, on the whole, the pharmacodynamic index results of rats in each administration group were in the order of Flu group, D group > F group, E group > C group > A group, which indicates that the antidepressant effect of the extract in D group is comparable to that of Flu group medicine, and the antidepressant effect of the extract in A group is the worst.

To sum up, the extracts in A group, C group, D group, E group and F group can play an antidepressant role by resisting HPA axis hyperfunction, increasing monoamine neurotransmitter content and improving neuronal plasticity. On the whole, the antidepressant effect of the extract in D group is comparable to that from Flu group medicine, and the antidepressant effect of the extract in A group is the worst. There were significant differences between D group and E group.

Table 9 Table of the results by order of pharmacodynamic indices of rats in each group

| Pharmacodynamic indices | Order of each group |
| --- | --- |
| Body weight gain | Flu group>D group>F group>E group>C group> A group |
| Percentage of sugar water consumption | Flu group>D group>E group>F group>C group>A group |
| Scores of horizontal movements | Flu group>D group>E group>F group>C group>A group |
| Scores of vertical movements | Flu group>D group>E group>F group>C group>A group |
| Content of CORT in the plasma | Flu group>D group>F group>E group>C> group>A group |
| Contents of DA, NE and 5-HT in the cortex | Flu group>D group>F group>E group>C group>A group |
| BDNF content in the cortex | D group>Flu group>F group>E group>C group>A group |
| Contents of DA, NE and 5-HT in the hippocampus | Flu group>D group>F group>E group>C group>A group |
| BDNF content in the hippocampus | D group>Flu group>F group>E group>C group>A group |
| Total | Flu group, D group>E group, F group>C group>A group |

Test example II Comparative data of the composition extracted in separate groups and the composition extracted together in the present application

1 Experimental animals and materials

1.1 Experimental animals

90 SPF male Sprague Dawley(SD) rats [purchased from China Institute of Food and Drug Control, license number: SCXK (Beijing) 2017-0005], weighing 180 ~ 200 g, were adaptively raised and tested in the Experimental Animal Center of Tianjin University of Traditional Chinese Medicine. Feeding conditions: $(22\pm2)$ °C, humidity $(50\pm10)\%$, natural light, free access to food and water. The feeding and operating rules of experimental animals comply with the relevant regulations of Tianjin University of Traditional Chinese Medicine on feeding and use of experimental animals.

1.2 Drugs and reagents

B group (Example 14), C group, D group and F group are the same as those in Table 1 of test example I.
1.3 Experimental equipment and instruments: same as those in the test example I.

2 Experimental methods

2.1 Preparation of drug solution

2.1.1 Preparation of fluoxetine suspension

Fluoxetine hydrochloride capsules (20 mg/ capsule) were taken and the shell of the capsule was removed, and 0.5% CMC-Na solution was added therein, grinded and formulated into 1 mg/mL of fluoxetine suspension.

2.1.2 Preparation of drug solution in each group

The daily clinical dosages of the extracts from B group, C group, D group and F group were 3.26 g, 3.37 g, 3.23 g and 2.72 g, respectively, and the doses for the rat converted according to 15 times the clinically equivalent extracts dose was 4.4010 g/kg, 4.5495 g/kg, 4.3605 g/kg, 3.6720 g/kg, respectively.

2.2 Establishment of CUMS combined with solitary depression model

After 90 SD rats were raised adaptively for 7 days, the open-box experiment was carried out in a quiet environment. The rats were screened according to the principle that the scores of horizontal movement and vertical movement were similar, and the rats with too high or too low scores were excluded. Screened rats were randomly divide into normal group, model group, Flu group, B group, C group, D group and F group, with 12 rats in each group.
The rats in the normal group were raised normally with four rats per cage, with free access to water and food, and without receiving any other additional stimulation. The rest of the rats in each group were raised in a single cage, and received the following two different stress stimuli randomly every day, and the same stimuli cannot appear continuously, so that the rats cannot predict what kind of stimuli and avoid their adaptability. CUMS stimulation factors include: restraint for 2 h, ice water platform for 2 h, noise stimulation (110 dB, 30 min), plantar electric shock (current 4 mA, 50 times, with an interval of 5 s each time), hot drying in the oven at 45°C for 10 min, inversion of day and night for 24 h, wet bedding for 12 h, and tail clamping (about 1 cm from the tail tip of rats for 2 min). Rats in each group were continuously modeled, and their body weight gain, the percentage of sugar water consumption, and the scores of horizontal and vertical movements in the open-box experiment were measured every week.

2.3 Measurement of pharmacodynamic indices

2.3.1 Behavioral experiments

(1) Body weight change

The body weight of the rats was measured on days 0, 7, 14, 21, 28, 35, 42, and 54 of the experiment, respectively. The body weight of the rats in each group was recorded, and the results of weekly body weight changes of the rats in each group during the modeling process and the body weight gain (%) before and after modeling were analyzed. The formula is as follows:

$$\text{Weight gain(\%)} = \frac{(\text{Weight on day } 54 - \text{Weight on day } 0)}{\text{Weight on day } 0} \times 100\%$$

(2) Sugar water consumption experiment

The sugar water consumption experiments were conducted on the rats in each group on days 0, 7, 14, 21, 28, 35, 42, and 54 of the experiment. Three days before the experiment, sugar water adaptation was carried out. Two bottles of 1% sucrose solution were placed on the cage for 24 h, followed by one bottle of 1% sucrose solution and one bottle of pure water for 24 h. The positions of the water bottles were changed in the morning and afternoon, and water and food were forbidden for 24 h on day 3. In the formal experiment, one bottle of 1% sucrose solution and one bottle of pure water were placed for the rats in each group. The positions of the water bottles were changed in the morning and afternoon. The amount of sucrose solution (the amount of sugar water consumption) and the amount of pure water consumption within 24 h for rats in each group were recorded, and the percentage of sugar water consumption (%) was calculated. The formula is as follows:

$$\text{the percentage of sugar water consumption}(\%)$$
$$= \frac{\text{the amount of sugar water consumption}}{(\text{the amount of sugar water consumption} + \text{the amount of pure water consumption})} \times 100\%$$

(3) Open-box experiment

The rats in each group were subjected to open-box experiment on days 0, 7, 14, 21, 28, 35, 42, and 54 of the experiment. The size of the open box used in the experiment is 100 cm×100 cm×50 cm (length× width× height), and the inner side is black. The bottom surface is divided into 100(10 cm×10 cm) small squares of the same size with white lines. Each rat was placed in a fixed square in the center in turn. The activity route of rats within 3 minutes without human interference was recorded. The number of the experimental rats crossing the small squares on the bottom surface was taken as the score of horizontal movement. Every time the rat crosses one square (entering with more than 3 claws), it was counted as one point for horizontal movement. If the experimental rats walked along a straight line, it was counted as one point for every 10 cm. The number of times the experimental rats stood upright was taken as the score of vertical movement, and the separation of both forelimbs from the bottom surface of the open box is the mark of vertical movement. No matter how long the rats stood, the laying down of both forelimbs was taken as the vertical movement score of 1 point. After each rat was tested, the open box was thoroughly cleaned and then the next rat was tested. The scores of horizontal and vertical movements of each rat were recorded, and the differences in the scores of horizontal and vertical movements of the rats in each group were analyzed.

2.3.2 Detection of biochemical indicators

(1) Acquisition of materials

After the behavioral experiment, about 1.5 mL of blood was taken from the rat's orbit, and placed in a centrifuge tube soaked with heparin at 8000 r·min$^{-1}$ at 4 °C for 10 min. The supernatant was taken and placed in a refrigerator at -80 °C for storage for the determination of corticosterone (CORT) level in the plasma of rat.

After blood was taken from the orbit, the neck was removed and killed. The cortex and hippocampus were quickly peeled off on the ice surface, weighed, placed in a freezing tube and quickly frozen in liquid nitrogen, and then transferred to a refrigerator at -80 °C for storage. The contents of nerve growth nutrition factor (BDNF), 5-hydroxytryptamine (5-HT), dopamine (DA) and norepinephrine (NE) in the cortex and hippocampus of rats were determined.

(2) Determination and calculation of CORT, BDNF, 5-HT, DA and NE

Enzyme linked immunosorbent assay (ELISA) was used to determine the content of CORT in the plasma, and the contents of BDNF, 5-HT, DA and NE in the cortex and hippocampus of rats in each group, and the absorbance (OD value) of each well was measured in sequence at 450 nm wavelength. A standard curve was plotted with the content of standard substance as abscissa and OD value as ordinate, and obtained a regression equation. The OD values of CORT in the plasma and BDNF, 5-HT, DA and NE in the cortex and hippocampus of rats in each group are substituted into the regression equation, and the contents of CORT in the plasma and BDNF, 5-HT, DA and NE in the cortex and hippocampus of rats in each group are calculated.

2.4. Experimental data processing and statistical analysis

The experimental data were expressed as $\bar{x} \pm s$ and were analyzed by SPSS 22.0 statistical software. The content of CORT in the plasma and the contents of BDNF, 5-HT, DA and NE in the cortex and hippocampus of rats were analyzed for inter-group difference analysis by One-way ANOVA.

3 Experimental results and analysis

3.1 Results of behavioral experiments

3.1.1 Weight change results

Different degrees of body weight gain were observed for rats in each experimental group as the modeling process proceeds, and the results of body weight gain of rats in each group are shown in Table 10. The body weight gain of the rats in the model group was decreased significantly as compared with that in the normal group (P<0.01), which indicated that CUMS combined with the solitary modeling method affected the appetite of rats, resulted in slow body weight gain, and simulated the symptoms of anorexia and weight loss in the patients with depression.

Table 10 Comparison of body weight gain of rats in each group ($\overline{x}\pm s$, n=10

| Group | Dosage(g/kg) | Weight on day 0(g) | Weight on day 54(g) | Body weight gain(%) |
|---|---|---|---|---|
| Normal group | - | 222.30±7.18 | 402.9±20.02 | 81.40±10.61 |
| Model group | - | 222.00±11.47 | 286.8±15.10▼ | 29.49±9.34▼ |
| B group | 4.40 | 221.50±7.50 | 349.4±17.26# | 57.81±7.42# |
| C group | 4.55 | 219.90±8.44 | 314.70±9.56# | 43.52±5.84# |
| D group | 4.36 | 221.30±6.06 | 367.30±14.66# | 66.14±9.16# |
| F group | 3.67 | 219.60±7.34 | 332.00±9.79# | 51.29±5.42# |
| Flu group | 0.01 | 227.70±8.38 | 372.90±12.64# | 67.67±8.73# |
| Compared with the normal group, ∇P<0.05, ▼P < 0.01; compared with the model group, * p < 0.05, # P<0.01 | | | | |

The body weight gain of rats in each administration group was significantly higher than that of the model group (P<0.01), the body weight gain of rats in the Flu group was higher than that in the D group, but there was no significant difference in the body weight gain between the Flu group and the D group (P>0.05), and there were significant differences in the body weight gain of rats in other groups (P<0.05), indicating that each administration group can resist the body weight loss of CUMS combined with solitary depression model rats, and thus improve the symptoms of anorexia and weight loss of CUMS combined with solitary depression model rats. From Table 10, it can be seen that the body weight gain of rats in each administration group is in the order of Flu group, D group > B group > F group > C group, which indicates that the effect of extract in D group on improving the symptoms of anorexia and weight loss of rats with CUMS combined with solitary depression model is comparable to Flu group medicine, and the effect of extract in C group on improving the symptoms of anorexia and weight loss of rats with CUMS combined with solitary depression model is the worst. There were significant differences between D group and B group (P<0.01).

3.1.2 Results of the percentage of sugar water consumption

The percentage of sugar water consumption of rats in each experimental group was decreased gradually as the modeling process proceeds, and the results of the percentage of sugar water consumption of rats in each group are shown in Table 11. The percentage of sugar water consumption of rats in the model group was decreased significantly as compared with that in the normal group (P<0.01), which indicated that CUMS combined with solitary modeling method reduced the percentage of sugar water consumption in rats, and simulated the symptoms of anhedonia in the patients with depression.

Table 11 Results of the percentage of sugar water consumption of rats in each group ($\overline{x}\pm s$, n=10)

| Group | Dosage(g/kg) | On day 0(%) | On day 54(%) |
|---|---|---|---|
| Normal group | - | 90.29±3.58 | 93.88±3.59 |
| Model group | - | 89.52±3.65 | 65.03±3.82▼ |
| B group | 4.40 | 91.41±3.58 | 85.00±4.65# |
| C group | 4.55 | 89.72±5.77 | 72.57±3.19# |
| D group | 4.36 | 91.42±3.24 | 90.05±5.30# |
| F group | 3.67 | 89.61±3.72 | 82.63±5.12# |
| Flu group | 0.01 | 89.37±3.64 | 91.64±4.99# |
| Compared with the normal group, ∇P<0.05, ▼P < 0.01; compared with the model group, * p < 0.05, # P<0.01 | | | |

The percentage of sugar water consumption in each administration group was increased significantly as compared with that in the model group (P<0.01), and the percentage of sugar water consumption of rats in Flu group was higher than that in D group, but there was no significant difference between the percentages of sugar water consumption of rats in Flu group and D group (P>0.05). The percentage of sugar water consumption of rats in B group was higher than that in F group, but there was no significant difference between the percentages of sugar water consumption of rats in B group and F group (P>0.05), and the percentages of sugar water consumption of rats in other groups all had significant difference (P<0.05), which indicated that the symptoms of anhedonia of rats with CUMS combined with solitary depression were improved in each administration group. From Table 11, it can be seen that the relationships of the percentages of sugar water consumption of rats in each administration group are in the order of Flu group, D group > B group, F group > C group , which indicated that the effect of the extract in D group on improving the symptoms of anhedonia of rats with CUMS combined with solitary depression model is similar to that in Flu group medicine, and the effect of the extract in C group on improving the symptoms of anhedonia of rats with CUMS combined with solitary depression model is the worst. There were significant differences between D group and B group (P<0.05).

3.1.3 Results of the open-box experiment

(1) Score of horizontal movement

The scores of open-box horizontal movement of rats in each experimental group decreased gradually as the modeling process proceeds, and the results of the scores of open-box horizontal movement of rats in each group are shown in Table 12. The scores of open-box horizontal movement of rats in the model group decreased significantly as compared with those in the normal group (P<0.01), which indicated that CUMS combined with solitary modeling method can significantly reduce the emotion and autonomous activity of rats, and simulate the symptoms of autonomous hypoactivity in the patients with depression.

Table 12 Results of the scores of horizontal movement of rats in each group in open-box experiment ($\bar{x}\pm s$, n=10)

| Group | Dosage(g/kg) | On day 0(%) | On day 54(%) |
|---|---|---|---|
| Normal group | - | 184.60±10.82 | 151.30±11.24 |
| Model group | - | 185.10±9.98 | 71.70±10.86▼ |
| B group | 4.40 | 182.60±11.85 | 118.50±13.62# |
| C group | 4.55 | 182.20±9.37 | 84.40±11.06# |
| D group | 4.36 | 184.90±10.35 | 133.50±13.07# |
| F group | 3.67 | 182.90±9.69 | 113.20±7.96# |
| Flu group | 0.01 | 185.80±11.20 | 138.2±17.38# |
| Compared with the normal group, ▽P<0.05, ▼P < 0.01; compared with the model group, * p < 0.05, # P<0.01 | | | |

The scores of open-box horizontal movement in the administration group were significantly higher as compared with those in the model group (P<0.01). The scores of open-box horizontal movement of rats in Flu group were higher than those in D group, but there was no significant difference between the scores of open-box horizontal movement of rats in Flu group and D group (P>0.05), and there were significant differences between the scores of open-box horizontal movement of rats in other groups (P<0.05), which indicated that each administration group can effectively up-regulate the emotion and autonomous hypoactivity of rats with CUMS combined with solitary depression model, and thus improve the symptoms of autonomous hypoactivity of rats with CUMS combined with solitary depression model. From Table 12, it can be seen that the relationships of the scores of horizontal movement of rats in each administration group are in the order of Flu group, D group > B group > F group > C group, which indicated that the effect of the extract in D group on improving the symptoms of hypoactivity of rats with CUMS combined with solitary depression model is equivalent to that in Flu group medicine, and the effect of the extract in C group on improving the symptoms of hypoactivity of rats with CUMS combined with solitary depression model is the worst. There were significant differences between D group and B group (P<0.01).

(2) Score of vertical movement

The scores of vertical and horizontal movements of rats in each experimental group decreased gradually as the modeling process proceeds, and the results of the scores of open-box vertical movements of rats in each group are shown in Table 14. The scores of open-box vertical movement of rats in the model group decreased significantly as compared with those in the normal group (P<0.01), which indicated that CUMS combined with solitary modeling method can significantly reduce the spatial exploration ability of rats, and simulate the symptoms of reduced curiosity of the patients with depression about the fresh environment.

Table 14 Results of the scores of vertical movements of rats in each group in open-box experiment ($\bar{x}\pm s$, n=10)

| Group | Dosage(g/kg) | On day 0 | On day 54 |
|---|---|---|---|
| Normal group | - | 15.60±1.35 | 13.90±1.52 |
| Model group | - | 16.10±1.66 | 4.40±1.62▼ |
| B group | 4.40 | 16.00±1.76 | 9.40±1.26# |
| C group | 4.55 | 16.30±1.06 | 5.90±1.20* |
| D group | 4.36 | 15.80±1.62 | 10.80±1.32# |
| F group | 3.67 | 15.80±1.75 | 8.10±1.20# |
| Flu group | 0.01 | 16.30±1.89 | 12.20±1.55# |
| Compared with the normal group, ▽P<0.05, ▼P < 0.01; compared with the model group, * P < 0.05, # P<0.01 |||||

The scores of open-box vertical movement in each administration group were significantly higher than those in the normal group (P<0.05), and there were significant differences between of scores of open-box vertical movement of rats in other groups (P<0.05), which indicated that each administration group can improve the symptoms of reduced curiosity of rats with CUMS combined with solitary depression model about fresh environment. From Table 14, it can be seen that the relationships of the scores of vertical movements of rats in each administration group were in the order of Flu group > D group > B group > F group > C group, which indicates that the effect of the extract in D group on improving the symptoms of reduced curiosity of rats with CUMS combined with solitary depression model about the fresh environment is second only to Flu group medicine, and the effect of the extract in C group on improving the symptoms of reduced curiosity of rats with CUMS combined with solitary depression model about the fresh environment is the worst. There were significant differences between D group and B group (P<0.05).

3.2 Test results of biochemical indicators

3.2.1 Comparison of the contents of CORT in the plasma of rats in each group

Corticosterone (CORT) is a glucocorticoid. High concentration of Corticosterone combines with its receptor to produce neurotoxicity, resulting in neuronal damage in the hippocampus, and resulting in hyperfunction of hypothalamus-pituitary-adrenal (HPA) axis, thereby causing depression.

The contents of CORT in the plasma of rats in each group are shown in Table 15. The contents of CORT in the plasma of rats in the model group increased significantly as compared with those in the normal group (P<0.01), which indicated that CUMS combined with solitary modeling method significantly increased the CORT contents in the plasma of rats, resulting in HPA axis hyperfunction and thus depression in rats.

Table 15 Comparison of the contents of CORT in the plasma of rats in each group ($\bar{x}\pm s$, n=10)

| Group | Dosage(g/kg) | CORT(ng/L) |
|---|---|---|
| Normal group | - | 314.1979±9.3951 |
| Model group | - | 460.6571±9.3572▼ |
| B group | 4.40 | 362.5143± 21.6609# |
| C group | 4.55 | 398.7368±15.0062# |
| D group | 4.36 | 340.6571±21.1191# |
| F group | 3.67 | 374.7254±20.8706# |
| Flu group | 0.01 | 330.6143±9.0210# |
| Compared with the normal group, ▽P<0.05, ▼P < 0.01; compared with the model group, * P < 0.05, # P<0.01 ||||

The contents of CORT in the plasma of rats in each administration group decreased significantly as compared with those in the model group (P<0.01), and there were significant differences between the contents of CORT in the plasma of rats in other groups (P<0.05), which indicated that the HPA axis hyperfunction of rats with depression of CUMS combined with solitary model can be improved in each administration group, and thus an antidepressant role can be exerted. From Table 15, it can be seen that the relationships of the contents of CORT in the plasma of rats in each administration group were in the order of Flu group < D group < B group < F group < C group, which indicated that the antidepressant effect of the extract in D group is second only to Flu group medicine, and the antidepressant effect of the extract in C group is the worst. There were significant differences between D group and B group (P<0.05).

3.2.2 Comparison of the contents of DA, NE, 5-HT and BDNF in the cortex and hippocampus of rats in each group

Dopamine (DA), norepinephrine (NE) and 5-hydroxytryptamine (5-HT) are monoamine neurotransmitters in the central nervous system of the brain. Brain-derived neurotrophic factor (BDNF) is a protein with nutritional support to neurons, and the decreased level of BDNF can result in depression.

The contents of DA, NE, 5-HT and BDNF in the cortex of rats in each group are shown in Table 16. The comparison of contents of DA, NE, 5-HT and BDNF in the hippocampus of rats in each group are shown in Table 17. The contents of DA, NE, 5-HT and BDNF in the cortex and hippocampus of rats in the model group were significantly decreased as compared with the normal group (P<0.01), which indicated that CUMS combined with the solitary modeling method significantly reduced the contents of monoamine neurotransmitters DA, NE, 5-HT and the contents of brain-derived neurotrophic factor BDNF in the cortex and hippocampus of rats, thereby resulting in depression in rats.

Table 16 Comparison of the contents of DA, NE, 5-HT and BDNF in the cortex of rats in each group ($\bar{x}\pm s$, n=10

| Group | Dosage (g/kg) | DA(ng/g) | NE(ng/g) | 5-HT(ng/g) | BDNF(ng/g) |
|---|---|---|---|---|---|
| Normal group | - | 0.3285±0.0007 | 0.6685±0.0357 | 1.4087±0.1803 | 2.7776±0.1319 |
| Model group | - | 0.3133±0.0017▼ | 0.5062±0.0426▼ | 1.1012±0.0879▼ | 1.8309±0.0657▼ |
| B group | 4.40 | 0.3272±0.0010# | 0.5877±0.0155# | 1.2412±0.0983# | 2.6347±0.05727# |
| C group | 4.55 | 0.3192±0.0020# | 0.5475±0.0383# | 1.1563±0.0263 * | 2.2252±0.0066# |
| D group | 4.36 | 0.3277±0.0010# | 0.6394±0.0251# | 1.2438±0.1108# | 2.7249±0.0320# |
| F group | 3.67 | 0.3254±0.0009# | 0.5863±0.0279# | 1.1940±0.0441 * | 2.5777±0.0389# |
| Flu group | 0.01 | 0.3281±0.0007# | 0.6403±0.0213# | 1.2966±0.1235# | 2.7404±0.0100# |
| Compared with the normal group, ∇P<0.05, ▼P < 0.01; compared with the model group, * P< 0.05, # P<0.01 | | | | | |

Table 17 Comparison of the contents of DA, NE, 5-HT and BDNF in the hippocampus of rats in each group ($\bar{x}\pm s$, n=10)

| Group | Dosage (g/kg) | DA (ng/g) | NE (ng/g) | 5-HT (ng/g) | BDNF (ng/g) |
|---|---|---|---|---|---|
| Normal group | - | 0.4617±0.0003 | 0.7854±0.0331 | 1.8432±0.2812 | 5.2704±0.0546 |
| Model group | - | 0.3601±0.0010▼ | 0.5934±0.0437▼ | 1.1326±0.1982▼ | 2.3596±0.0099▼ |
| B group | 4.40 | 0.4397±0.0029# | 0.7372±0.0300# | 1.5201±0.2123# | 4.4966±0.0464# |
| C group | 4.55 | 0.3910±0.0142# | 0.6604±0.0443# | 1.3015±0.0843# | 2.9849±0.2012# |
| D group | 4.36 | 0.4410±0.0026# | 0.7562±0.0252# | 1.6096±0.1587# | 4.9267±0.0395# |
| F group | 3.67 | 0.4312±0.0005# | 0.7126±0.0267# | 1.4721±0.1260# | 4.2171±0.0373# |
| Flu group | 0.01 | 0.4530±0.0018# | 0.7701±0.0363# | 1.7504±0.2315# | 5.1440±0.0329# |
| Compared with the normal group, ∇P<0.05, ▼P < 0.01; compared with the model group, * P< 0.05, # P<0.01 | | | | | |

The contents of DA in the cortex and hippocampus of rats in each administration group were increased significantly as compared with those in the model group (P<0.01). There were no significant differences between the contents of DA in the cortex of rats in D group and B group (P>0.05), and there were significant differences in the contents of DA in the cortex and hippocampus of rats in other groups (P<0.05), which indicated that in each administration group, the contents of DA in the cortex and hippocampus of rats with CUMS combined with solitary depression model can be improved, and thus an antidepressant role was exerted. From Table 15-16, it can be seen that the relationships of DA contents in the cortex of rats in each administration group were in the order of Flu group > D group, B group > F group > C group, and there were significant differences between B group and D group (P>0.05).

The relationships of the contents of DA in the hippocampus of rats in each administration group were in the order of Flu group > D group > B group > F group > C group, and there were significant differences between D group and B group (P<0.01). The above results showed that the antidepressant effect of the extract in D group was second only to that of the medicine in Flu group medicine, and the antidepressant effect of the extract in C group was the worst.

The contents of NE in the cortex and hippocampus of rats in each administration group were increased significantly as compared with those in the model group (P<0.01), but there were no significant differences in the contents of NE in the cortex and hippocampus of rats between Flu group and D group (P>0.05), and there were significant differences in the contents of NE in the cortex and hippocampus of rats among other groups (P<0.05), which indicated that in each administration group, the contents of NE in the cortex and hippocampus of rats with CUMS combined with solitary depression model can be increased, thus exerting anti-depression effect. From Table 15-16, it can be seen that the relationships of the contents of NE in the cortex and hippocampus of rats in each administration group were in the order of Flu group, D group > B group > F group > C group, and there were significant differences between D group and B group. The results showed that the antidepressant effect of the extract in D group was comparable to that of the medicine from Flu group medicine, and the antidepressant effect of the extract in C group was the worst.

The contents of 5-HT in the cortex and hippocampus of rats in each administration group were increased significantly as compared with the model group (P<0.05), but there were no significant differences between the contents of 5-HT in the cortex of rats in D group and B group (P>0.05), and there were significant differences in the contents of 5-HT in the cortex and hippocampus of rats in other groups (P<0.05), which indicated that in each administration group, the contents of 5-HT in the cortex and hippocampus of rats with CUMS combined with solitary depression model can be increased, and thus an antidepressant role was exerted. From Table 15-16, it can be seen that the relationships of the 5-HT contents in the cortex of rats in each administration group were in the order of Flu group > D group, B group > F group > C group, and there were significant differences between B group and D group (P<0.05). The relationships of 5-HT contents in the hippocampus of rats in each administration group were in the order of Flu group > D group > B group, F group > C group, and there were significant differences between D group and B group (P<0.05). The results showed that the antidepressant effect of the extract in D group was second only to that the medicine from Flu group medicine, and the antidepressant effect of the extract in C group was the worst.

The BDNF contents in the cortex and hippocampus of rats in each administration group were increased significantly as compared with the model group (P<0.01). There were no significant differences in the contents of BDNF in the cortex of rats between Flu group and D group (P>0.05), and there were significant differences in the contents of BDNF in the cortex and hippocampus of rats in other groups (P<0.05), which indicated that in each administration group, the contents of BDNF in the cortex and hippocampus of rats with CUMS combined with solitary depression model were increased and the repair and regeneration of neurons were promoted, and thus an antidepressant role was exerted. From table 15-16, it can be seen that the relationships of the contents of BDNF in the cortex of rats in each administration group were in the order of Flu group, D group > B group > F group > C group, and the relationships of the contents of NE in the cortex and hippocampus of rats in each administration group were in the order of Flu group > D group > B group > F group > C group, and there were significant differences between D group and B group (P<0.01), which indicated that the antidepressant effect of the extract in D group was second only to that of the medicine from Flu group medicine, and the antidepressant effect of the extract in C group was the worst.

4. Summary

4.1 Establishment of depression model rats

After the depression model of rat was established by chronic unpredictable mild stress (CUMS) combined with solitary method, the behavioral indices (body weight change, percentage of sugar water consumption, scores of exercise in the open-box experiment) of rats in the model group were significantly lower than those of rats in the normal group. Biochemical indices DA, NE, 5-HT and BDNF were significantly lower than those in the normal group, while CORT was significantly higher than those in the normal group, which indicated that the depression model of rats was successfully established.

4.2 Behavioral and biochemical indices measurement results

The behavioral indices (body weight gain, percentage of sugar water consumption, score of exercise in the open-box experiment) and biochemical indices (DA, NE, 5-HT, BDNF) of rats in each administration group were significantly higher as compared with the model group, while the CORT in the plasma was significantly lower than that in the model group, which indicated that all administration groups had antidepressant effects.

The results of the order of behavioral and biochemical indices of rats in each administration group are shown in Table 18. It can be seen from the table that the extract of D group has the best antidepressant effect, while the extract of C group has the worst antidepressant effect.

Table 18 Table of the order of results of behavioral and biochemical indices of rats in each group

| Pharmacodynamic index | | The order of each group |
|---|---|---|
| | Body weight gain | Flu group, D group>B group>F group>C group |
| behavioral indices | Percentage of sugar water consumption | Flu group, D group>B group, F group>C group |
| | Scores of horizontal movements | Flu group, D group>B group>F group>C group |
| | Scores of vertical movements | Flu group>D group>B group>F group>C group |
| biochemical indices | CORT content in the plasma | Flu group<D group<B group<F group<C group |
| | DA content in the cortex | Flu group>D group, B group>F group>C group |
| | DA content in the hippocampus | Flu group>D group>B group>F group>C group |
| | NE content in the cortex and hippocampus | Flu group, D group>B group, F group>C group |
| | 5-HT content in the cortex | Flu group>D group, B group>F group>C group |
| | 5-HT content in the hippocampus | Flu group>D group>B group, F group>C group |
| | BDNF content in the cortex | Flu group, D group>B group>F group>C group |
| | BDNF content in the hippocampus | Flu group>D group>B group>F group>C group |

To sum up, after the depression model of rats was successfully established in this study, although there were no significant differences in the measurement results of behavioral indices and biochemical indices among some groups, on the whole, the behavioral indices and biochemical indices in the cortex and hippocampus of rats in each administration group were in the order of Flu group > D group > B group > F group > C group, and the contents of CORT in the plasma were in the order of Flu group < D group < B group < F group < C group.

The extracts in D group, B group, F group and C group can exert antidepressant effects by resisting HPA axis hyperfunction, increasing the content of monoamine neurotransmitters and improving neuronal plasticity. The behavioral indices and biochemical indices of extracts in groups D and B were higher than those of F group; the general trend of D group and B group is that D group > B group, but there were no significant differences in the contents of 5-HT and DA in the cortex, and other indices are significantly different between D group and B group; the general trend of B group and F group is that B group > F group, but there were no significant differences in the percentages of sugar water consumption, the contents of NE in the cortex and hippocampus, and the contents of 5-HT in the hippocampus. The extract in C group has the worst antidepressant effect.

Example 32: Investigation on grouping methods of the five medicinal materials Experimental objective: the Chinese Thorowax Root *(Bupleuri Radix)* component was found to interfere with the quantification of ferulic acid in Chinese Angelica *(Angelicae Sinensis Radix)* during the quality control of the extracts obtained by hydration of the five medicinal materials. To solve this problem, the grouping extraction was specially designed to avoid the interference.

Experimental methods: the grouping extraction of the five medicinal materials was designed based on three principles: 1. Chinese Thorowax Root *(Bupleuri Radix)* was separated from Chinese Angelica *(Angelicae Sinensis Radix)* to avoid the interference of Chinese Thorowax Root *(Bupleuri Radix)* component on the quantification of ferulic acid in Chinese Angelica *(Angelicae Sinensis Radix);* 2. at least one stable index component is contained in the extracts in each group to characterize the process stability from the extract to the formulation; 3, aromatic water was collected in that extraction process, and the aromatic water was added into the extract at the end point of concentration, so that the whole extract is close to the components of the traditional applied water decoction.

Experimental results:

1. Grouping method from the perspective of oil collection:

After the prescription is reduced, there are five ingredients in the prescription: Chinese Thorowax Root *(Bupleuri Radix),* Chinese Angelica *(Angelicas Sinensis Radix),* Atractylodis Macrocephalae Rhizoma (processed with bran), liauorice root *(Radix Glycyrrhizae)* and White Peony Root *(Paeoniae Alba Radix),* among which Chinese Thorowax Root *(Bupleuri Radix),* Chinese Angelica *(Angelicas Sinensis Radix),* and Atractylodis Macrocephalae Rhizoma (processed with bran) are the volatile ingredients. Investigation on the oiling-out for single component: Chinese Thorowax Root *(Bupleuri Radix)* yielded very little oil, which could not be collected, about 0.2 ml/kg; Atractylodis Macrocephalae Rhizoma (processed with bran) had a small amount of oil, 0.95 ml/kg; Chinese Angelica *(Angelicas Sinensis Radix)* yielded more oil, 4.3 ml/kg.

From the perspective of volatile oil collection, Chinese Angelica *(Angelicas Sinensis Radix)* and Atractylodis Macrocephalae Rhizoma (processed with bran) can collect volatile oil together, and Chinese Thorowax Root *(Bupleuri Radix)* will be combined with water extraction because it has very little oil.

2. Grouping method from the perspective of analyzing Marker:

Objective: to screen for suitable analytical markers in the group of Chinese Angelica *(Angelicas Sinensis Radix)* and Atractylodis Macrocephalae Rhizoma (processed with bran) to characterize the stability of the process from the extract to the formulation.

Chinese Angelica *(Angelicas Sinensis Radix):* The ligustilide and ferulic acid screened were not suitable for use as the analytical marker. Ligustilide was a volatile component, and due to the influence of the collection status of volatile oil, the fluctuation between the extraction batches was large, and the stability risk of the volatile components was high. The content of ferulic acid was low (about 0.1%) and the property of ferulic acid was unstable (sensitive to high temperature and light). Ferulic acid can be quantified in the extract, but it could not be accurately quantified due to interference from Chinese Thorowax Root *(Bupleuri Radix)* in the preparation.

Atractylodis Macrocephalae Rhizoma (processed with bran): atractylone and atractylenolide III components screened are not suitable as the analytical marker. Atractylone is a volatile component, and due to the influence of the collection status of volatile oil, the fluctuation between the extraction batches was large, and the stability risk of the volatile components was high. The level of the content of atractylenolide III is low (about 0.01%), and the detectability in the preparation is poor, so there is a risk in the transfer rate from the extract to the formulation.

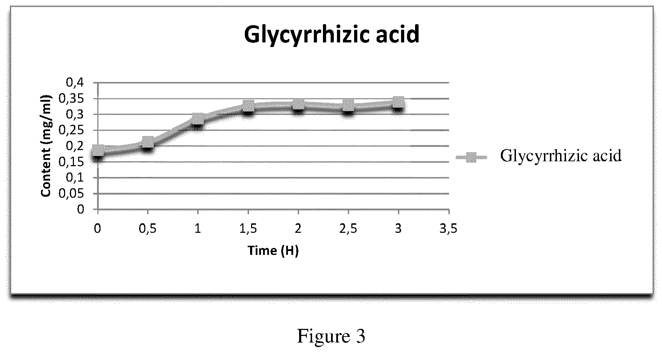

It is considered to add liauorice root *(Radix Glycyrrhizae)* into the group of Chinese Angelica *(Angelicas Sinensis Radix)* and Atractylodis Macrocephalae Rhizoma (processed with bran), and the glycyrrhizic acid component will be used as an analytical marker of the extracts in this group to characterize the process stability from the extracts to the formulation. As the nature of glycyrrhizic acid was relatively stable and it was not affected by long-term decocting, as shown in Fig. 3, and the prescription proportion of glycyrrhizic acid is low, which is beneficial to batch alignment between two groups of extracts.

Experimental conclusion:

The extraction groups determined after the experimental investigation were as follows: Chinese Thorowax Root *(Bupleuri Radix)* and White Peony Root *(Paeoniae Alba Radix)* were combined and extracted to obtain *Xiaoyao Chaibai* extract; Chinese Angelica *(Angelicae Sinensis Radix),* Atractylodis Macrocephalae Rhizoma (processed with bran) and prepared liauorice root *(Glycyrrhizae Radix et Rhizon Praeparata cum Melle)* were combined and extracted to obtain *Xiaoyao Dangbaigan* extract. As shown in Fig. 2, the specificity of content analysis method of ferulic acid component in the *Xiaoyao Dangbaigan* extract containing Chinese Angelica *(Angelicae Sinensis Radix)* is qualified, and comprehensive quality control of the extract can be achieved. As shown in Fig. 3, the stable glycyrrhizic acid component can be used as an analytical marker of the extract in this group to characterize the process stability from the extract to the formulation.

**Claims**

1. A traditional Chinese medicine composition with the effects of relieving mental stress and resisting depression, which is prepared from the formulation of Chinese medicine raw materials in parts by weight consisting of Chinese Thorowax Root *(Bupleuri Radix),* White Peony Root *(Paeoniae Alba Radix),* Atractylodis Macrocephalae Rhizoma (processed with bran), Chinese Angelica *(Angelicas Sinensis Radix),* prepared liauorice root *(Glycyrrhizae Radix et Rhizon Praeparata cum Melle)* and optionally with or without Indian Bread *(Poria),* wherein the formulation without the Indian Bread *(Poria)* is formulation 1:

   10-30 parts of Chinese Thorowax Root *(Bupleuri Radix),*
   10-30 parts of White Peony Root *(Paeoniae Alba Radix),*
   15-30 parts of Atractylodis Macrocephalae Rhizoma (processed with bran),
   10-30 parts of Chinese Angelica *(Angelicas Sinensis Radix)* and
   15-24 parts of prepared liauorice root *(Glycyrrhizae Radix et Rhizon Praeparata cum Melle),*
   wherein the formulation with Indian Bread *(Poria)* is formulation 2:

   10-30 parts of Chinese Thorowax Root *(Bupleuri Radix),*
   10-30 parts of White Peony Root *(Paeoniae Alba Radix),*
   15-30 parts of Atractylodis Macrocephalae Rhizoma (processed with bran),
   10-30 parts of Chinese Angelica *(Angelicas Sinensis Radix),*
   10-30 parts of Indian Bread *(Poria)* and
   15-24 parts of prepared liauorice root *(Glycyrrhizae Radix et Rhizon Praeparata cum Melle).*

2. The traditional Chinese medicinal composition according to claim 1, wherein the formulation 1 is:

   15-25 parts of Chinese Thorowax Root *(Bupleuri Radix),*
   15-25 parts of White Peony Root *(Paeoniae Alba Radix),*
   15-25 parts of Atractylodis Macrocephalae Rhizoma (processed with bran),
   15-25 parts of Chinese Angelica *(Angelicas Sinensis Radix),* and
   15-20 parts of prepared liauorice root *(Glycyrrhizae Radix et Rhizon Praeparata cum Melle),*
   wherein the formulation 2 is

   15-25 parts of Chinese Thorowax Root *(Bupleuri Radix),*
   15-25 parts of White Peony Root *(Paeoniae Alba Radix),*
   15-25 parts of Atractylodis Macrocephalae Rhizoma (processed with bran),
   15-25 parts of Chinese Angelica *(Angelicas Sinensis Radix),*
   15-25 parts of Indian Bread *(Poria),* and
   15-20 parts of prepared liauorice root *(Glycyrrhizae Radix et Rhizon Praeparata cum Melle).*

3. The traditional Chinese medicine composition according to claim 1, wherein the formula 1 is:

   20 parts of Chinese Thorowax Root *(Bupleuri Radix),*
   20 parts of White Peony Root *(Paeoniae Alba Radix),*
   20 parts of Atractylodis Macrocephalae Rhizoma (processed with bran),
   20 parts of Chinese Angelica *(Angelicas Sinensis Radix),* and
   16 parts of prepared liauorice root *(Glycyrrhizae Radix et Rhizon Praeparata cum Melle);*
   wherein the formulation 2 is

   20 parts of Chinese Thorowax Root *(Bupleuri Radix),*
   20 parts of White Peony Root *(Paeoniae Alba Radix),*
   20 parts of Atractylodis Macrocephalae Rhizoma (processed with bran),
   20 parts of Chinese Angelica *(Angelicas Sinensis Radix),*
   20 parts of Indian Bread *(Poria),* and
   16 parts of prepared liauorice root *(Glycyrrhizae Radix et Rhizon Praeparata cum Melle).*

4. The traditional Chinese medicine composition according to any one of claims 1-3, wherein the traditional Chinese medicine composition is prepared by a method comprising the following steps: weighing the medicinal materials according to the formulation ratio, adding water of 4-7 times weight of the medicinal materials, extracting for 1-3 times, each time for 1-3 h, filtering after extraction, combining the filtrates from the two extractions, standing for 4h or above, taking the supernatant, and concentrating the supernatant to obtain an extract; taking the extract as the active component of the medicine, and adding medicinal excipients when necessary, to prepare into a traditional Chinese medicine composition pharmaceutical formulation;

preferably, the traditional Chinese medicine composition is prepared by a method comprising the following steps: weighing the medicinal materials according to the formulation ratio, adding water of 6-7 times weight, extracting for 2-3h, filtering, adding water of 5-6 times weight into the medicine residue, extracting for 1h, filtering, combining the filtrates from the two extractions, standing for 4h or above, taking the supernatant, concentrating, controlling the concentration endpoint at the sugar content of 65%-70%, taking the extract as the active component of the medicine and adding medicinal excipients when necessary, and making into a traditional Chinese medicine composition pharmaceutical formulation.

5. The traditional Chinese medicine composition according to any one of claims 1-3, wherein the traditional Chinese medicine composition of formulation 1 comprises 9 parts of *Xiaoyao Chaibai* extract and 32 parts of *Xiaoyao Dangbaigan* extract by dry weight; wherein the dry content of saikosaponin B2 is 0.63-10.0 mg/g and the dry content of paeoniflorin is 9.4-115.9 mg/g in the *Xiaoyao Chaibai* extract; the dry content of glycyrrhizic acid is 1.4 ~26.1mg/g, the dry content of ferulic acid is 0.13~0.91mg/g, and the dry content of atractylenolide III is 0.0038~0.30mg/g in the *Xiaoyao Dangbaigan* extract; the *Xiaoyao Chaibai* extract is extracted from Chinese Thorowax Root *(Bupleuri Radix)* and White Peony Root *(Paeoniae Alba Radix);* and the *Xiaoyao Dangbaigan* extract is extracted from Atractylodis Macrocephalae Rhizoma (processed with bran), Chinese Angelica *(Angelicae Sinensis Radix)* and prepared liauorice root *(Glycyrrhizae Radix et Rhizon Praeparata cum Melle).*

6. The traditional Chinese medicine composition according to claim 5, wherein the dry content of saikosaponin B2 is 0.94~6.8mg/g, and the dry content of paeoniflorin is 17.6~110.9 mg/g in the *Xiaoyao Chaibai* extract; the dry content of glycyrrhizic acid is 6.9~26.1mg/g, the dry content of ferulic acid is 0.16~0.79mg/g, and the dry content of atractylenolide III is 0.0047-0.25mg/g in the *Xiaoyao Dangbaigan* extract.

7. The traditional Chinese medicine composition according to claim 5, wherein the traditional Chinese medicine composition is prepared by a method comprising the following steps:

(1) extraction of *Xiaoyao Chaibai* extract: adding water of 4-7 times weight into Chinese Thorowax Root *(Bupleuri Radix)* and White Peony Root *(Paeoniae Alba Radix)* medicinal materials, extracting for 1-3 times, each time for 1-3 h, filtering after extraction, combining the filtrates from the two extractions, standing for 4h or above, taking the supernatant, and concentrating the supernatant to obtain the *Xiaoyao Chaibai* extract;
(2) the extraction of the *Xiaoyao Dangbaigan* extract: adding water of 4-7 times weight into Chinese Angelica *(Angelicae Sinensis Radix),* Atractylodis Macrocephalae Rhizoma (processed with bran) and prepared liauorice root *(Glycyrrhizae Radix et Rhizon Praeparata cum Melle)* medicinal materials, extracting for 1-3 times, each time for 1-3 h, collecting the volatile oil and aromatic water after the extraction, filtering the extracted solution after the extraction, combining the extracted solutions from the two extractions, standing for 4 h or above, taking the supernatant, concentrating, adding the collected volatile oil and aromatic water, and concentrating again to obtain the *Xiaoyao Dangbaigan* extract;
(3) taking and evenly mixing the *Xiaoyao Chaibai* extract and the *Xiaoyao Dangbaigan* extract according to the proportion, to prepare the traditional Chinese medicine composition.

8. The traditional Chinese medicine composition according to claim 7, wherein the traditional Chinese medicine composition is prepared by a method comprising the following steps:

(1) extraction of *Xiaoyao Chaibai* extract: adding water of 6 times weight into Chinese Thorowax Root *(Bupleuri Radix)* and White Peony Root *(Paeoniae Alba Radix)* medicinal materials, extracting for 2h, filtering, adding water of 5 times weight into the medicine residue, extracting for 1h, filtering, combining the filtrates from the two extractions, standing for 4h or above, taking the supernatant, concentrating, and controlling the density of the concentration endpoint to be 1.26-1.28 to obtain the *Xiaoyao Chaibai* extract;
(2) the extraction of the *Xiaoyao Dangbaigan* extract: adding water of 6 times weight into Chinese Angelica *(Angelicas Sinensis Radix),* Atractylodis Macrocephalae Rhizoma (processed with bran) and prepared liauorice root *(Glycyrrhizae Radix et Rhizon Praeparata cum Melle)* medicinalmaterials, extracting for 2h, filtering, adding water of 5 times weight to the medicine residues, extracting for 1h, collecting volatile oil and aromatic water after extraction, filtering the extracted solution, combining the extracted solutions from the two extractions, standing for 4h or above, taking the supernatant, concentrating, and concentrating to an end point, adding the collected volatile oil and aromatic water, concentrating again to an endpoint, and controlling the density of the concentration endpoint to be 1.29-1.31;

(3) taking and evenly mixing the *Xiaoyao Chaibai* extract and the *Xiaoyao Dangbaigan* extract according to the proportion, to prepare the traditional Chinese medicine composition.

**9.** The traditional Chinese medicine composition according to claim 5, wherein the traditional Chinese medicine composition is prepared by a method comprising the following steps:

(1) extraction of *Xiaoyao Chaibai* extract: adding water of 4-7 times weight into Chinese Thorowax Root *(Bupleuri Radix)* and White Peony Root *(Paeoniae Alba Radix)* medicinal materials, extracting for 1-3 times, each time for 1-3 h, filtering after extraction, combining the filtrates from the two extractions, standing for 4h or above, taking the supernatant, and concentrating to obtain the *Xiaoyao Chaibai* extract;

(2) the extraction of the *Xiaoyao Dangbaigan* extract: adding water of 4-7 times weight into Chinese Angelica *(Angelicas Sinensis Radix),* Atractylodis Macrocephalae Rhizoma (processed with bran) and prepared liauorice root *(Glycyrrhizae Radix et Rhizon Praeparata cum Melle)* medicinal materials, extracting for 1-3 times, each time for 1-3 h, collecting the volatile oil and aromatic water after the extraction, filtering the extracted solution after the extraction, combining the extracted solutions from the two extractions, standing for 4 h or above, taking the supernatant, concentrating, adding the collected volatile oil and aromatic water, and concentrating again to obtain the *Xiaoyao Dangbaigan* extract;

(3) taking and evenly mixing the *Xiaoyao Chaibai* extract and the *Xiaoyao Dangbaigan* extract according to the proportion, taking the Xiaoyao Chaibai extract and the Xiaoyao dangbai gan extract as medicinal active components, and adding medicinal excipients when necessary, to prepare the traditional Chinese medicine composition pharmaceutical formulation;

preferably comprising the following steps:

extraction of *Xiaoyao Chaibai* extract: adding water of 6 times weight into Chinese Thorowax Root *(Bupleuri Radix)* and White Peony Root *(Paeoniae Alba Radix)* medicinal materials, extracting for 2h, filtering, adding water of 5 times weight into the medicine residue, extracting for 1h, filtering, combining the filtrates from the two extractions, standing for 4h or above, taking the supernatant, concentrating, and controlling the density of the concentration endpoint to be 1.26-1.28 to obtain the *Xiaoyao Chaibai* extract;

the extraction of the *Xiaoyao Dangbaigan* extract: adding water of 6 times weight into Chinese Angelica *(Angelicas Sinensis Radix),* Atractylodis Macrocephalae Rhizoma (processed with bran) and prepared liauorice root *(Glycyrrhizae Radix et Rhizon Praeparata cum Melle)* medicinal materials, extracting for 2h, filtering, adding water of 5 times weight in the medicine residues, extracting for 1h, collecting volatile oil and aromatic water after extraction, filtering the extracted solution, combining the extracted solutions from the two extractions, standing for 4h or above, taking the supernatant, concentrating, and concentrating to an endpoint, adding the collected volatile oil and aromatic water, concentrating again to an endpoint, and controlling the density of the concentration endpoint to be 1.29-1.31;

taking and evenly mixing the *Xiaoyao Chaibai* extract and the *Xiaoyao Dangbaigan* extract according to the proportion, taking the *Xiaoyao Chaibai* extract and the *Xiaoyao Dangbaigan* extract as the active components of the medicine, and adding medicinal excipients when necessary to prepare the traditional Chinese medicine composition pharmaceutical formulation.

**10.** The traditional Chinese medicine composition according to claim 4 or 9, wherein the traditional Chinese medicine composition is in the form of tablets, capsules, granules, oral liquid, dripping pills or pills.

Figure 1

Figure 2

Figure 3

## INTERNATIONAL SEARCH REPORT

International application No.

**PCT/CN2021/109326**

| A. | CLASSIFICATION OF SUBJECT MATTER |
|---|---|

A61K 36/71(2006.01)i; A61K 36/484(2006.01)i; A61K 36/284(2006.01)i; A61K 36/233(2006.01)i; A61K 36/232(2006.01)i; A61K 36/076(2006.01)i; A61P 25/24(2006.01)i

According to International Patent Classification (IPC) or to both national classification and IPC

| B. | FIELDS SEARCHED |
|---|---|

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, SIPOABS, CNMED, CNTXT, DWPI, VEN, CNKI, PUBMED, 读秀, 超星, 百度, E药全库 抑郁, 紧张, 精神, 情绪, 逍遥散, 逍遥丸, 提取, 水, 柴胡, 白芍, 白术, 当归, 甘草, 茯苓, 芍药苷, 甘草酸, Depress+, nervous, spirit, feelings, xiaoyao powder, xiaoyao pill, extract+, water, Radix Bupleuri, Radix Paeoniae Alba, Rhizoma Atractylodis Macrocephalae, Radix Angelicae Sinensis, Radix Glycyrrhizae, Poria, paeoniflorin, glycyrrhizic acid

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 104644776 A (LIANG, Benfu) 27 May 2015 (2015-05-27)<br>description, embodiment 1 | 1-10 |
| Y | CN 101732427 A (GUANGZHOU BAIYUNSHAN HUTCHISON WHAMPOA TRADITIONAL CHINESE MEDICINE CO., LTD.) 16 June 2010 (2010-06-16)<br>claims 1-12, description paragraphs 0032-0034 | 1-10 |
| Y | 许腾 等 (XU, Teng et al.). "复方柴归方抗抑郁作用剂量筛选及药效验证 ( Dose Screening and Efficacy Verification of Antidepressant Effect of Compound Chaigui Prescription)"<br>山西大学学报 (自然科学版) (Journal of Shanxi University (Natural Science Edition)),<br>Vol. 43, No. 1, 31 March 2020 (2020-03-31),<br>page 180 left-hand column paragraph 5 | 1-10 |

☑ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 October 2021** | **22 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/109326**

**C.    DOCUMENTS CONSIDERED TO BE RELEVANT**

| Category* | Citation of document, with indication, where appropriate, of the relevant passages | Relevant to claim No. |
|---|---|---|
| Y | 夏小涛 (XIA, Xiaotao). "复方柴归方治疗轻中度抑郁症临床疗效观察 (Clinical Observation on Treatment of Mild to Moderate Depression by Compound Chaigui Formula of TCM)" <br> 中国优秀硕士学位论文全文数据库（电子期刊），医药卫生科技辑 (China Doctoral Dissertations Full-Text Database, Electronic Journals), Medicine & Public Health), <br> No. 3, 15 March 2018 (2018-03-15), <br> E056-569, page 18 table 3.2, page 57 paragraph 4— page 59 paragraph 4 | 1-10 |
| Y | 邓红 等 (DENG, Hong et al.). "不同提取工艺对柴胡-白芍药对药效的影响 (Influences of Different Extraction Technologies on Pharmacodynamic Action of Radix Bupleuri Matched Paeoniae Alba)" <br> 食品与药品 (Food and Drug), Vol. 14, No. 9, 15 September 2012 (2012-09-15), <br> abstract, page 315 right-hand column paragraphs 2-3, page 316 right-hand column paragraphs 1-2 | 1-10 |
| Y | 王晖 (WANG, Hui). "薄荷醇对柴胡镇痛作用的影响 (non-official translation: Effect of Menthol on the Analgesic Effect of Radix Bupleuri)" <br> 时珍国药研究 (ShiZhen Journal of Traditional Chinese Medicine Research), <br> Vol. 7, No. 4, 10 August 1996 (1996-08-10), <br> abstract | 1-10 |

Form PCT/ISA/210 (second sheet) (January 2015)

**INTERNATIONAL SEARCH REPORT**
Information on patent family members

International application No.

**PCT/CN2021/109326**

| Patent document cited in search report | | | Publication date (day/month/year) | Patent family member(s) | | | Publication date (day/month/year) |
|---|---|---|---|---|---|---|---|
| CN | 104644776 | A | 27 May 2015 | None | | | |
| CN | 101732427 | A | 16 June 2010 | CN | 101732427 | B | 12 December 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108653405 A
- CN 107625813 A
- CN 101732427 A
- CN 102813906 A
- CN 104644776 A
- CN 1017342427 A
- CN 104189833 A
- CN 102908600 A

**Non-patent literature cited in the description**

- Chinese Pharmacopoeia. 2015, 1354-1355